(19) **Europäisches Patentamt / European Patent Office / Office européen des brevets**

(11) **EP 4 235 202 B1**

(12) # EUROPÄISCHE PATENTSCHRIFT

(45) Veröffentlichungstag und Bekanntmachung des Hinweises auf die Patenterteilung:
**10.12.2025 Patentblatt 2025/50**

(21) Anmeldenummer: **22158527.6**

(22) Anmeldetag: **24.02.2022**

(51) Internationale Patentklassifikation (IPC):
*G01R 33/56* (2006.01)    *A61B 5/055* (2006.01)
*A61B 5/00* (2006.01)    *G06N 3/02* (2006.01)
*G06N 3/08* (2023.01)    *G06N 3/04* (2023.01)
*A61B 8/00* (2006.01)    *A61B 8/08* (2006.01)
*A61B 6/00* (2024.01)    *A61B 6/08* (2006.01)
*A61K 49/10* (2006.01)    *G06T 7/00* (2017.01)

(52) Gemeinsame Patentklassifikation (CPC):
**G06N 3/084; A61B 5/00; A61B 5/055; A61B 5/4244; A61B 5/7267; G01R 33/5601; G01R 33/5608; G06T 7/0016;** A61B 6/032; A61B 6/481; A61B 6/52; A61B 8/481; A61B 8/52; G06N 3/0442; G06N 3/0455;     (Forts.)

(54) **VORHERSAGE EINER REPRÄSENTATION EINES UNTERSUCHUNGSBEREICHS EINES UNTERSUCHUNGSOBJEKTS IN EINEM ZUSTAND EINER FOLGE VON ZUSTÄNDEN**

PREDICTION OF A REPRESENTATION OF AN OBJECT TO BE EXAMINED IN A STATE OF A SEQUENCE OF STATES

PRÉDICTION D'UNE REPRÉSENTATION D'UNE ZONE D'EXAMEN D'UN OBJET D'EXAMEN DANS UN ÉTAT D'UNE SÉQUENCE D'ÉTATS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**30.08.2023 Patentblatt 2023/35**

(73) Patentinhaber: **Bayer Aktiengesellschaft 51373 Leverkusen (DE)**

(72) Erfinder:
- **LENGA, Matthias**
  **24114 Kiel (DE)**
- **SCHÜTZ, Gunnar**
  **13467 Berlin (DE)**
- **VÖLKENING, Stephan**
  **12161 Berlin (DE)**

(74) Vertreter: **BIP Patents**
**c/o Bayer Intellectual Property GmbH**
**Alfred-Nobel-Straße 50**
**40789 Monheim am Rhein (DE)**

(56) Entgegenhaltungen:
**WO-A1-2021/052896**

- **ZHANG LING ET AL: "Spatio-Temporal Convolutional LSTMs for Tumor Growth Prediction by Learning 4D Longitudinal Patient Data", IEEE TRANSACTIONS ON MEDICAL IMAGING, IEEE, USA, vol. 39, no. 4, 25 September 2019 (2019-09-25), pages 1114 - 1126, XP011780999, ISSN: 0278-0062, [retrieved on 20200401], DOI: 10.1109/TMI.2019.2943841**

(52) Gemeinsame Patentklassifikation (CPC): (Forts.)
G06N 3/0464; G06N 3/047; G06N 3/0475;
G06N 3/0499; G06T 2207/10096;
G06T 2207/20081; G06T 2207/20084;
G06T 2207/30056

## EP 4 235 202 B1

**Beschreibung**

Die vorliegende Erfindung betrifft das technische Gebiet der Radiologie, insbesondere der Unterstützung von Radiologen bei radiologischen Untersuchungen mit Methoden der künstlichen Intelligenz. Die vorliegende Erfindung befasst sich mit dem Trainieren eines Modells des maschinellen Lernens und der Nutzung des trainierten Modells zur Vorhersage von Repräsentationen eines Untersuchungsbereich in einem oder mehreren Zuständen einer Folge von Zuständen bei einer radiologischen Untersuchung.

L. Zhang et al. offenbaren räumlich-zeitliche Faltungs-LSTMs für die Vorhersage von Tumorwachstum durch Lernen von 4D-Längsschnittdaten von Patienten (Spatio-Temporal Convolutional LSTMs for Tumor Growth Prediction by Learning 4D Longitudinal Patient Data, IEEE Trans Med Imaging. 2020 April ; 39(4): 1114-1126).

P. Luc et al. offenbaren Verfahren zur Vorhersage zukünftiger semantischer Segmentierungen (Predicting Deeper into the Future of Semantic Segmentation, Proceedings - 2017 IEEE International Conference on Computer Vision, ICCV 2017, 648-657).

Die zeitliche Verfolgung von Vorgängen innerhalb des Körpers eines Menschen oder eines Tieres mit bildgebenden Verfahren spielt unter anderem bei der Diagnose und/oder Therapie von Krankheiten eine wichtige Rolle.

Als ein Beispiel sei die Detektion und Differentialdiagnose fokaler Leberläsionen mittels dynamischer kontrastverstärkender Magnetresonanztomographie (MRT) mit einem hepatobiliären Kontrastmittel genannt.

Ein hepatobiliäres Kontrastmittel wie beispielsweise Primovist® kann zur Detektion von Tumoren in der Leber eingesetzt werden. Die Blutversorgung des gesunden Lebergewebes erfolgt in erster Linie über die Pfortader (Vena portae), während die Leberarterie (Arteria hepatica) die meisten Primärtumoren versorgt. Nach intravenöser Bolusinjektion eines Kontrastmittels lässt sich dementsprechend eine Zeitverzögerung zwischen der Signalanhebung des gesunden Leberparenchyms und des Tumors beobachten.

Neben malignen Tumoren findet man in der Leber häufig gutartige Läsionen wie Zysten, Hämangiome und fokal noduläre Hyperplasien (FNH). Für eine sachgerechte Therapieplanung müssen diese von den malignen Tumoren differenziert werden. Primovist® kann zur Erkennung gutartiger und bösartiger fokaler Leberläsionen eingesetzt werden. Es liefert mittels T1-gewichteter MRT Informationen über den Charakter dieser Läsionen. Bei der Differenzierung nutzt man die unterschiedliche Blutversorgung von Leber und Tumor und den zeitlichen Verlauf der Kontrastverstärkung.

Bei der durch Primovist® erzielten Kontrastverstärkung während der Anflutungsphase beobachtet man typische Perfusionsmuster, die Informationen für die Charakterisierung der Läsionen liefern. Die Darstellung der Vaskularisierung hilft, die Läsionstypen zu charakterisieren und den räumlichen Zusammenhang zwischen Tumor und Blutgefäßen zu bestimmen.

Bei T1-gewichteten MRT-Aufnahmen führt Primovist® 10-20 Minuten nach der Injektion (in der hepatobiliären Phase) zu einer deutlichen Signalverstärkung im gesunden Leberparenchym, während Läsionen, die keine oder nur wenige Hepatozyten enthalten, z.B. Metastasen oder mittelgradig bis schlecht differenzierte hepatozelluläre Karzinome (HCCs), als dunklere Bereiche auftreten.

Die zeitliche Verfolgung der Verteilung des Kontrastmittels bietet also eine gute Möglichkeit der Detektion und Differentialdiagnose fokaler Leberläsionen; allerdings zieht sich die Untersuchung über eine vergleichsweise lange Zeitspanne hin. Über diese Zeitspanne sollten Bewegungen des Patienten weitgehend vermieden werden, um Bewegungsartefakte in den MRT-Aufnahmen zu minimieren. Die lang andauernde Bewegungseinschränkung kann für einen Patienten unangenehm sein.

In der Offenlegungsschrift WO2021/052896A1 wird vorgeschlagen, eine oder mehrere MRT-Aufnahmen während der hepatobiliären Phase nicht messtechnisch zu erzeugen, sondern auf Basis von MRT-Aufnahmen aus einer oder mehreren vorangegangenen Phasen zu berechnen (vorherzusagen), um die Aufenthaltsdauer des Patienten im MRT-Scanner zu verkürzen

In dem in der Offenlegungsschrift WO2021/052896A1 beschriebenen Ansatz wird ein Modell des maschinellen Lernens trainiert, auf Basis von MRT-Aufnahmen eines Untersuchungsbereichs vor und/oder unmittelbar nach der Applikation eines Kontrastmittels eine MRT-Aufnahme des Untersuchungsbereichs zu einem späteren Zeitpunkt vorherzusagen. Das Modell wird also trainiert, mehrere MRT-Aufnahmen als Eingabedaten auf eine MRT-Aufnahme als Ausgabedaten abzubilden (engl. *mapping*).

Ausgehend vom beschriebenen Stand der Technik stellte sich die Aufgabe, die Vorhersagequalität des Modells des maschinellen Lernens zu verbessern und/oder ein Modell zu erstellen, das die Dynamik der Kontrastmittelverteilung lernt, um es vielfältig einsetzen zu können.

Diese Aufgabe wird durch die Gegenstände der vorliegenden unabhängigen Patentansprüche gelöst. Bevorzugte Ausführungsformen finden sich in den abhängigen Patentansprüchen sowie in der vorliegenden Beschreibung und in den Zeichnungen.

Ein erster Gegenstand ist ein computer-implementiertes Verfahren nach Anspruch 1.

Ein weiterer Gegenstand der vorliegenden Erfindung ist ein Computersystem nach Anspruch 9.

- eine Eingabeeinheit,

- eine Steuer- und Recheneinheit und

- eine Ausgabeeinheit,
  wobei die Steuer- und Recheneinheit konfiguriert ist,

- eine Repräsentation $R_p$ eines Untersuchungsbereichs zu empfangen, wobei die Repräsentation $R_p$ den Untersuchungsbereich in einem Zustand $Z_p$ einer Folge von Zuständen $Z_1$ bis $Z_n$ repräsentiert, wobei $p$ eine ganze Zahl ist, die kleiner als $n$ ist, wobei $n$ eine ganze Zahl ist, die größer als zwei ist,

- die Repräsentation $R_p$ einem trainierten Modell des maschinellen Lernens zuzuführen, wobei das Modell des maschinellen Lernens auf Basis von Trainingsdaten trainiert worden ist,

Ein weiterer Gegenstand der vorliegenden Erfindung ist ein Computerprogrammprodukt nach Anspruch 10.

Ein weiterer Gegenstand der vorliegenden Erfindung ist eine Verwendung eines Kontrastmittels in einem radiologischen Untersuchungsverfahren nach Anspruch 11.

Ein weiterer Gegenstand der vorliegenden Erfindung ist ein Kit nach Anspruch 12.

Die Erfindung wird nachstehend näher erläutert, ohne zwischen den Erfindungsgegenständen (Verfahren, Computersystem, Computerprogrammprodukt, Verwendung, Kit) zu unterscheiden. Die nachfolgenden Erläuterungen sollen vielmehr für alle Erfindungsgegenstände in analoger Weise gelten, unabhängig davon, in welchem Kontext (Verfahren, Computersystem, Computerprogrammprodukt, Verwendung, Kit) sie erfolgen.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar

Mit Hilfe der vorliegenden Erfindung können Repräsentationen eines Untersuchungsbereichs eines Untersuchungsobjekts vorhergesagt werden.

Erfindungsgemäß ist der Untersuchungsbereich die Leber oder ein Teil der Leber eines Menschen.

Der Untersuchungsbereich, auch Aufnahmevolumen (engl.: *field of view,* FOV) genannt, stellt insbesondere ein Volumen dar, welches in radiologischen Aufnahmen abgebildet wird. Der Untersuchungsbereich wird typischerweise durch einen Radiologen, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden.

Eine Repräsentation des Untersuchungsbereichs ist erfindungsgemäß eine radiologische Aufnahme.

Die "Radiologie" ist das Teilgebiet der Medizin, das sich mit der Anwendung vorwiegend elektromagnetischer Strahlen und (unter Einbezug etwa der Ultraschalldiagnostik) mechanischer Wellen zu diagnostischen, therapeutischen und/oder wissenschaftlichen Zwecken befasst. Neben Röntgenstrahlen kommen auch andere ionisierende Strahlungen wie Gammastrahlung oder Elektronen zum Einsatz. Da ein wesentlicher Einsatzzweck die Bildgebung ist, werden auch andere bildgebende Verfahren wie die Sonografie und die Magnetresonanztomographie (Kernspintomographie) zur Radiologie gerechnet, obwohl bei diesen Verfahren keine ionisierende Strahlung zum Einsatz kommt. Der Begriff "Radiologie" im Sinne der vorliegenden Erfindung umfasst damit insbesondere die folgenden Untersuchungsmethoden: Computertomografie, Magnetresonanztomografie, Sonografie.

In einer bevorzugten Ausführungsform der vorliegenden Erfindung handelt es sich bei der radiologischen Untersuchung um eine magnetresonanztomographische Untersuchung.

Die Magnetresonanztomographie, abgekürzt MRT oder MRI (engl. MRI: *Magnetic Resonance Imaging*), ist ein bildgebendes Verfahren, das vor allem in der medizinischen Diagnostik zur Darstellung von Struktur und Funktion der Gewebe und Organe im menschlichen oder tierischen Körper eingesetzt wird

Bei der MR-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten MR-Daten liegen zunächst als Rohdaten im Frequenzraum vor, und können durch anschließende inverse Fourier-Transformation in den Ortsraum (Bildraum) transformiert werden.

Eine Repräsentation des Untersuchungsbereichs im Sinne der vorliegenden Erfindung kann eine MRT-Aufnahme, ein Computertomogramm, ein Ultraschallbild oder dergleichen sein.

Eine Repräsentation des Untersuchungsbereichs im Sinne der vorliegenden Erfindung kann eine Repräsentation im Ortsraum (Bildraum) oder eine Repräsentation im Frequenzraum oder eine andere Repräsentation sein. Vorzugsweise liegen Repräsentationen des Untersuchungsbereichs in einer Ortsraum-Darstellung oder in einer Form vor, die sich in eine Ortsraum-Darstellung umwandeln (transformieren) lässt.

Eine Repräsentation im Sinne der vorliegenden Erfindung zeigt den Untersuchungsbereich in einem Zustand einer Folge von Zuständen.

Bei der Folge von Zuständen handelt es sich vorzugsweise um eine zeitliche Folge. Dabei können in einer zeitlichen Folge unmittelbar aufeinander folgende Zustände stets denselben zeitlichen Abstand zueinander aufweisen oder sie können variierende zeitliche Abstände aufweisen. Mischformen sind ebenfalls denkbar.

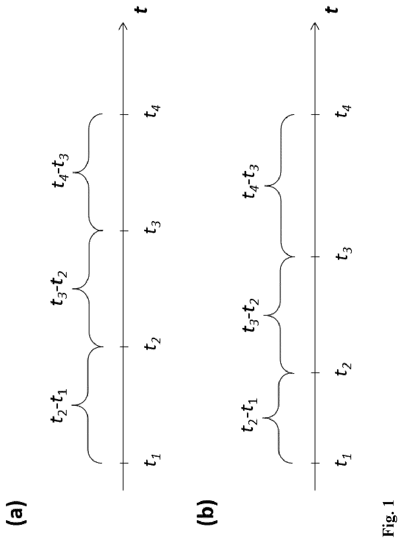

Dies sei anhand von Fig. 1 erläutert.

Fig. 1 zeigt zwei Zeitstrahlen ($t$ = Zeit), einen ersten Zeitstrahl (a) und einen zweiten Zeitstrahl (b). Auf den Zeitstrahlen sind jeweils definierte Zeitpunkt $t_1$, $t_2$, $t_3$ und $t_4$ markiert. An jedem Zeitpunkt kann der Untersuchungsbereich einen anderen Zustand aufweisen, d.h. jeder Zeitpunkt kann einen Zustand des Untersuchungsbereichs repräsentieren.

Die Zeitpunkte $t_1$, $t_2$, $t_3$ und $t_4$ bilden für jeden Zeitstrahl eine zeitliche Folge: $t_1 \rightarrow t_2 \rightarrow t_3 \rightarrow t_4$. Der Zeitpunkt $t_2$ folgt unmittelbar auf den Zeitpunkt $t_1$, der zeitliche Abstand zwischen $t_1$ und $t_2$ beträgt $t_2$-$t_1$; der Zeitpunkt $t_3$ folgt unmittelbar auf den Zeitpunkt $t_2$, der zeitliche Abstand zwischen $t_2$ und $t_3$ beträgt $t_3$-$t_2$; der Zeitpunkt $t_4$ folgt unmittelbar auf den Zeitpunkt $t_3$, der zeitliche Abstand zwischen den Zeitpunkten $t_3$ und $t_4$ beträgt $t_4$-$t_3$.

Im Fall des ersten Zeitstrahls (a) sind die zeitlichen Abstände von unmittelbar aufeinander folgenden Zeitpunkten für alle unmittelbar aufeinander folgenden Zeitpunkte gleich: $t_2$-$t_1$ = $t_3$-$t_2$ = $t_4$-$t_3$.

Im Fall des zweiten Zeitstrahls (b) variieren die zeitlichen Abstände von unmittelbar aufeinander folgenden Zeitpunkten für alle unmittelbar aufeinander folgenden Zeitpunkte: $t_2$-$t_1 \neq t_3$-$t_2 \neq t_4$-$t_3$. Im vorliegenden Beispiel steigen die zeitlichen Abstände im Fall der zweiten Zeitachse (b) unmittelbar aufeinander folgenden Zeitpunkte an: $t_2$-$t_1 < t_3$-$t_2 < t_4$-$t_3$. Es ist aber auch denkbar, dass sie abfallen oder zunächst ansteigen und dann abfallen oder zunächst abfallen und dann ansteigen oder anders entlang der Zeitachse verteilt sind.

Es sei angemerkt, dass die Zeitachse nicht zwangsläufig in Richtung zunehmender Zeit verlaufen muss. Das bedeutet, dass der Zeitpunkt $t_1$ von dem Zeitpunkt $t_2$ aus gesehen nicht zwingend in der Vergangenheit liegen muss; es ist auch denkbar, dass die Zeitachse eine abnehmende Zeit anzeigt und der Zeitpunkt $t_1$ vom Zeitpunkt $t_2$ aus gesehen in der Zukunft liegt. Mit anderen Worten: liegt eine zeitliche Folge von Zuständen vor, so kann ein unmittelbar auf einen ersten Zustand folgender zweiter Zustand vom Zeitpunkt des ersten Zustands aus betrachtet in der Zukunft liegen oder er kann in der Vergangenheit liegen.

Die (vorzugsweise zeitliche) Folge kann in einem ersten Schritt festgelegt werden. Die zeitliche Folge definiert, welche Trainingsdaten zum Trainieren des Modells des maschinellen Lernens verwendet werden und welche Repräsentationen das Modell des maschinellen Lernens erzeugen (vorhersagen) kann Mit anderen Worten; zum Trainieren des Modells des maschinellen Lernens sind Referenz-Repräsentationen des Untersuchungsbereichs in den Zuständen erforderlich, die durch die Folge von Zuständen definiert sind, und das trainierte Modell des maschinellen Lernens kann üblicherweise nur Repräsentationen von solchen Zuständen erzeugen, die Teil des Trainings gewesen sind (Ausnahmen von dieser Regel sind weiter hinten in der Beschreibung aufgeführt).

Erfindungsgemäß definiert die Folge von Zuständen verschiedene Zustände des Untersuchungsbereichs vor und/oder während und/oder nach einer oder mehreren Applikationen eines Kontrastmittels.

"Kontrastmittel" sind Substanzen oder Gemische von Substanzen, die die Darstellung von Strukturen und Funktionen des Körpers bei bildgebenden radiologischen Verfahren verbessern.

Beispiele für Kontrastmittel sind in der Literatur zu finden (siehe z.B. A. S. L. Jascinth et al.: Contrast Agents in computed tomography: A Review, Journal of Applied Dental and Medical Sciences, 2016, Vol. 2, Issue 2, 143 - 149; H. Lusic et al.: X-ray-Computed Tomography Contrast Agents, Chem. Rev. 2013, 113, 3, 1641-1666; https://www.radiology.wisc.edu/wp-content/uploads/2017/10/contrast-agents-tutorial.pdf, M. R. Nough *et al.: Radiographie and magnetic resonances contrast agents:* Essentials and tips, for safe practices, World J Radiol. 2017 Sep 28; 9(9): 339-349; L. C. Abonyi et al.: Intravascular Contrast Media in Radiography: Historical Development & Review ofRisk Factors for Adverse Reactions, South American Journal of Clinical Research, 2016, Vol. 3, Issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362).

Vorzugsweise handelt es sich bei dem Kontrastmittel um ein MR-Kontrastmittel. MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren. Superparamagnetische führen zu einer überwiegenden T2-Verkürzung, während paramagnetische Kontrastmittel im Wesentlichen zu einer T1-Verkürzung führen. Die Wirkung dieser Kontrastmittel ist indirekt, da das Kontrastmittel selbst kein Signal abgibt, sondern nur die Signalintensität in seiner Umgebung beeinflusst. Ein Beispiel für ein superparamagnetisches Kontrastmittel sind Eisenoxidnanopartikel (SPIO, engl.: *superparamagnetic iron oxide*). Beispiele für paramagnetische Kontrastmittel sind Gadolinium-Chelate wie Gadopentetat-Dimeglumin (Handelsname: Magnevist® u.a.), Gadotersäure (Dotarem®, Dotagita®, Cyclolux®), Gadodiamid (Omniscan®), Gadoteridol (ProHance®) und Gadobutrol (Gadovist®).

Erfindungsgemäß handelt es sich bei dem MR-Kontrastmittel um ein hepatobiliäres Kontrastmittel. Ein hepatobiliäres Kontrastmittel zeichnet sich dadurch aus, dass es spezifisch von Leberzellen, den Hepatozyten, aufgenommen wird, sich im Funktionsgewebe (Parenchym) anreichert und den Kontrast in gesundem Lebergewebe verstärkt. Ein Beispiel eines hepatobiliären Kontrastmittels ist das Dinatriumsalz der Gadoxetsäure (Gd-EOB-DTPA Dinatrium), das in dem US-Patent No. 6,039,931A beschrieben ist unter dem Markennamen Primovist® und Eovist® kommerziell erhältlich ist.

Nach der intravenösen Applikation eines hepatobiliären Kontrastmittels in Form eines Bolus in eine Armvene eines Menschen erreicht das Kontrastmittel die Leber zunächst über die Arterien. Diese sind in den entsprechenden MRT-Aufnahmen kontrastverstärkt dargestellt. Die Phase, in der die Leberarterien in MRT-Aufnahmen kontrastverstärkt dargestellt sind, wird als "arterielle Phase" bezeichnet

Anschließend erreicht das Kontrastmittel die Leber über die Lebervenen. Während der Kontrast in den Leberarterien bereits abnimmt, erreicht der Kontrast in den Lebervenen ein Maximum. Die Phase, in der die Lebervenen in MRT-Aufnahmen kontrastverstärkt dargestellt sind, wird als "portalvenöse Phase" bezeichnet

An die portalvenöse Phase schließt sich die "Übergangsphase" (engl. *transitional phase*) an, in der der Kontrast in den Leberarterien weiter absinkt, der Kontrast in den Lebervenen ebenfalls absinkt. Bei Verwendung eines hepatobiliären Kontrastmittels steigt der Kontrast in den gesunden Leberzellen in der Übergangsphase allmählich an.

Die arterielle Phase, die portalvenöse Phase und die Übergangsphase werden zusammenfassend auch als "dynamische Phase" bezeichnet.

10-20 Minuten nach seiner Injektion führt ein hepatobiliäres Kontrastmittel zu einer deutlichen Signalverstärkung im gesunden Leberparenchym. Diese Phase wird als "hepatobiliäre Phase" bezeichnet. Das Kontrastmittel wird aus den Leberzellen nur langsam ausgeschieden; dementsprechend kann die hepatobiliäre Phase zwei Stunden und mehr andauern.

Die genannten Phasen sind beispielsweise in den folgenden Publikationen näher beschrieben: J. Magn. Reson. Imaging, 2012, 35(3): 492-511, doi:10.1002/jmri.22833; Clujul Medical, 2015, Vol. 88 no. 4: 438-448, DOI: 10.15386/cjmed-414; Journal of Hepatology, 2019, Vol. 71: 534-542, http://dx.doi.org/10.1016/j.jhep.2019.05.005).

Bei den Zuständen der Folge der Zustände kann es sich beispielsweise um den Zustand der Leber als Untersuchungsbereich

- vor Applikation eines hepatobiliären Kontrastmittels,
- in der arteriellen Phase,
- in der portalvenösen Phase,
- in der Übergangsphase und/oder
- in der hepatobiliären Phase handeln.

Es ist denkbar, dass es mehr oder weniger Zustände gibt.

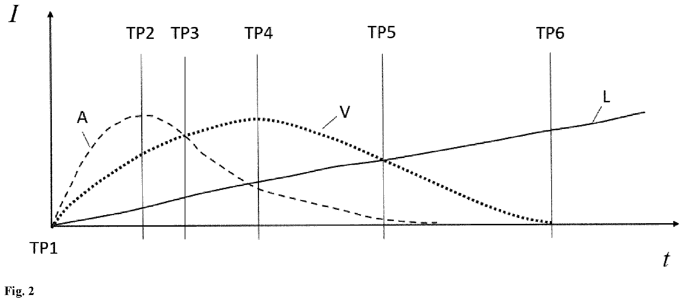

Die genannten Phasen werden nachfolgend anhand von Fig. 2 näher erläutert. Fig. 2 zeigt schematisch den zeitlichen Verlauf ($t$ = Zeit) der Signalinintensitäten $I$, die durch ein hepatobiliäres Kontrastmittel in Leberarterien A, Lebervenen V und gesunden Leberzellen L bei einer dynamischen kontrastverstärkten MRT-Untersuchung mit einem hepatobiliären Kontrastmittel hervorgerufen werden. Die Signalintensität $I$ korreliert positiv mit der Konzentration des Kontrastmittels in den genannten Bereichen. Bei einer intravenösen Bolusinjektion in den Arm eines Menschen steigt die Konzentration des Kontrastmittels in den Leberarterien A als erstes an (gestrichelte Kurve). Die Konzentration durchläuft ein Maximum und sinkt dann ab. Die Konzentration in den Lebervenen V steigt langsamer an als in den Leberarterien und erreicht ihr Maximum später (gepunktete Kurve). Die Konzentration des Kontrastmittels in den Leberzellen L steigt langsam an (durchgezogene Kurve) und erreicht ihr Maximum erst zu einem sehr viel späteren Zeitpunkt (in der Figur 2 nicht dargestellt). Es lassen sich einige charakteristische Zeitpunkte definieren: Zum Zeitpunkt TP1 wird Kontrastmittel intravenös als Bolus appliziert. Da die Applikation eines Kontrastmittels selbst eine gewisse Zeitspanne in Anspruch nimmt, definiert der Zeitpunkt TP1 vorzugsweise den Zeitpunkt, zu dem die Applikation abgeschlossen ist, Kontrastmittel also vollständig in das Untersuchungsobjekt eingebracht ist. Zum Zeitpunkt TP2 erreicht die Signalintensität des Kontrastmittels in den Leberarterien A ihr Maximum. Zum Zeitpunkt TP3 schneiden sich die Kurven der Signalintensitäten bei den Leberarterien A und den Lebervenen V. Zum Zeitpunkt TP4 durchläuft die Signalintensität des Kontrastmittels in den Lebervenen V ihr Maximum. Zum Zeitpunkt TP5 schneiden sich die Kurven der Signalintensitäten bei den Leberarterien A und den gesunden Leberzellen L. Zum Zeitpunkt TP6 sind die Konzentrationen in den Leberarterien A und den Lebervenen V auf ein Niveau gesunken, bei dem sie keine messbare Kontrastverstärkung mehr verursachen.

Die Zustände der Folge von Zuständen kann einen ersten Zustand umfassen, der vor dem Zeitpunkt TP1 vorliegt, einen zweiten Zustand, der zum Zeitpunkt TP2 vorliegt, einen dritten Zustand, der zum Zeitpunkt TP3 vorliegt, einen vierten Zustand, der zum Zeitpunkt TP4 vorliegt, einen fünften Zustand, der zum Zeitpunkt TP5 vorliegt und/oder einen sechsten Zustand, der zum Zeitpunkt TP6 und/oder danach vorliegt.

Allgemein kann

- ein erster Zustand der Zustand eines Untersuchungsbereichs zu einem ersten Zeitpunkt vor der Applikation eines Kontrastmittels sein,
- ein zweiter Zustand der Zustand des Untersuchungsbereichs zu einem zweiten Zeitpunkt, nach der Applikation des Kontrastmittels, sein,
- ein dritter Zustand der Zustand des Untersuchungsbereichs zu einem dritten Zeitpunkt, nach der Applikation des Kontrastmittels, sein,
- ein vierter Zustand ein Zustand des Untersuchungsbereichs zu einem vierten Zeitpunkt, nach der Applikation des Kontrastmittels, sein,

und so fort.

Dabei können unmittelbar aufeinander folgende Zeitpunkte wie oben beschrieben ganz oder teilweise einen gleichbleibenden zeitlichen Abstand zueinander aufweisen und/oder einen variablen zeitlichen Abstand zueinander aufweisen.

Erfindungsgemäß gibt es mindestens drei Zustände, vorzugsweise liegt die Zahl der Zustände zwischen 3 und 100. Die Zahl der Zustände ist jedoch nicht auf 100 beschränkt.

Zu jedem Zustand der Folge von Zuständen kann es eine oder mehrere Repräsentationen geben, die den Untersuchungsbereich in dem jeweiligen Zustand repräsentieren.

Üblicherweise gibt es zu einem ersten Zustand mindestens eine erste Repräsentation, die den Untersuchungsbereich in dem ersten Zustand repräsentiert, zu einem zweiten Zustand mindestens eine zweite Repräsentation, die den Untersuchungsbereich in dem zweiten Zustand repräsentiert, zu einem dritten Zustand mindestens eine dritte Repräsentation, die den Untersuchungsbereich in dem dritten Zustand repräsentiert, und so fort.

Zur besseren Verständlichkeit der vorliegenden Beschreibung wird die vorliegende Erfindung überwiegend auf Basis von jeweils einer Repräsentation pro Zustand beschrieben; dies soll jedoch nicht als eine Einschränkung der Erfindung verstanden werden. Der tatsächliche Schutzumfang wird ausschließlich durch die beigefügten Ansprüche definiert.

Zurückkommend auf das oben beschriebene Beispiel kann es beispielsweise

- eine oder mehrere erste Repräsentationen geben, die eine Leber vor der Applikation eines hepatobiliären Kontrastmittels repräsentierten,
- eine oder mehrere zweite Repräsentationen geben, die die Leber während der arteriellen Phase repräsentieren,
- eine oder mehrere dritte Repräsentationen geben, die die Leber während der portalvenösen Phase repräsentieren,
- eine oder mehrere vierte Repräsentationen geben, die die Leber während der Übergangsphase repräsentieren, und/oder
- eine oder mehrere fünfte Repräsentationen geben, die die Leber während der hepatobiliären Phase repräsentieren.

Mit Hilfe der vorliegenden Erfindung können eine oder mehrere Repräsentationen eines Untersuchungsbereichs eines Untersuchungsobjekts vorhergesagt werden, die den Untersuchungsbereich in einem Zustand einer Folge von Zuständen repräsentieren.

Dabei erfolgt die Vorhersage mit Hilfe eines Modells des maschinellen Lernens.

Ein "Modell des maschinellen Lernens" kann als eine computerimplementierte Datenverarbeitungsarchitektur verstanden werden. Das Modell kann Eingangsdaten empfangen und Ausgangsdaten auf der Grundlage dieser Eingangsdaten und Modell-Parametern liefern. Das Modell kann durch Training eine Beziehung zwischen den Eingabedaten und den Ausgabedaten erlernen. Beim Training können Modell-Parameter angepasst werden, um eine gewünschte Ausgabe für eine bestimmte Eingabe zu liefern.

Beim Trainieren eines solchen Modells werden dem Modell Trainingsdaten präsentiert, aus denen es lernen kann. Das trainierte Modell des maschinellen Lernens ist das Ergebnis des Trainingsprozesses. Die Trainingsdaten umfassen neben Eingabedaten die korrekten Ausgabedaten (Zieldaten), die das Modell auf Basis der Eingabedaten erzeugen soll. Beim Trainieren werden Muster erkannt, die die Eingabedaten auf die Zieldaten abbilden.

Im Trainingsprozess werden die Eingabedaten der Trainingsdaten in das Modell eingegeben, und das Modell erzeugt Ausgabedaten. Die Ausgabedaten werden mit den Zieldaten verglichen. Modell-Parameter werden so verändert, dass die Abweichungen zwischen den Ausgabedaten und den Zieldaten auf ein (definiertes) Minimum reduziert werden.

Die Abweichungen können mit Hilfe einer Fehlerfunktion (engl.: *loss function*) quantifiziert werden. Eine solche Fehlerfunktion kann verwendet werden, um einen Fehlerwert (engl.: *loss value*) für ein gegebenes Paar von Ausgabedaten und Zieldaten zu berechnen. Das Ziel des Trainingsprozesses kann darin bestehen, die Parameter des maschinellen Lernmodells so zu verändern (anzupassen), dass der Fehlerwert für alle Paare des Trainingsdatensatzes auf ein (definiertes) Minimum reduziert wird.

Handelt es sich bei den Ausgabedaten und den Zieldaten beispielsweise um Zahlen, kann die Fehlerfunktion die absolute Differenz zwischen diesen Zahlen sein. In diesem Fall kann ein hoher absoluter Fehlerwert bedeuten, dass ein oder mehrere Modell-Parameter in hohem Maße geändert werden müssen.

Bei Ausgabedaten in Form von Vektoren können beispielsweise Differenzmetriken zwischen Vektoren wie der mittlere quadratische Fehler, ein Kosinusabstand, eine Norm des Differenzvektors wie ein euklidischer Abstand, ein Tschebyscheff-Abstand, eine Lp-Norm eines Differenzvektors, eine gewichtete Norm oder jede andere Art von Differenzmetrik zweier Vektoren als Fehlerfunktion gewählt werden.

Bei höherdimensionalen Ausgaben, wie z.B. zweidimensionalen, dreidimensionalen oder höherdimensionalen Ausgaben, kann z.B. eine elementweise Differenzmetrik verwendet werden. Alternativ oder zusätzlich können die Ausgabedaten vor der Berechnung eines Fehlerwertes transformiert werden, z.B. in einen eindimensionalen Vektor.

Im vorliegenden Fall wird das Modell des maschinellen Lernens anhand von Trainingsdaten trainiert, eine Repräsentation eines Untersuchungsbereichs in einem Zustand einer Folge von Zuständen auf Basis von Repräsentationen des Untersuchungsbereichs in vorangegangenen Zuständen der Folge von Zuständen zu erzeugen, wobei das Modell ausgehend von einem ersten Zustand nacheinander Repräsentationen des Untersuchungsbereichs in nachfolgenden Zuständen erzeugt, wobei die Erzeugung jeder Repräsentation des Untersuchungsbereichs in jedem Zustand zumindest anteilig auf Basis einer (vorhergesagten) Repräsentation des vorangegangenen Zustands erfolgt.

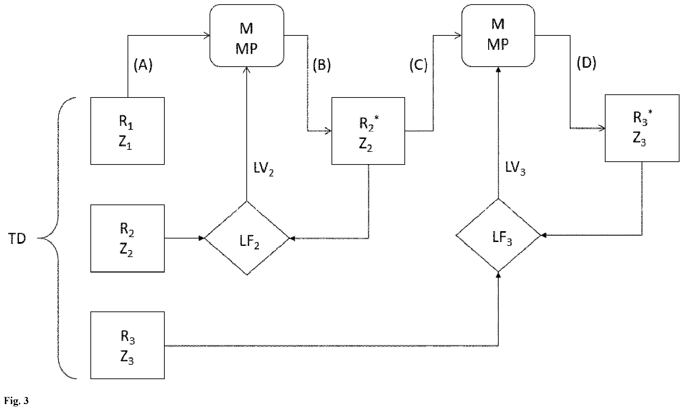

Dies sei anhand eines Beispiels von drei Zuständen erläutert, ohne die Erfindung auf diese Ausführungsform beschränken zu wollen. Bei dieser Erläuterung wird auf Fig. 3 Bezug genommen.

Fig. 3 zeigt drei Repräsentationen, eine erste Repräsentation $R_1$, eine zweite Repräsentation $R_2$ und eine dritte Repräsentation $R_3$. Die erste Repräsentation $R_1$ repräsentiert einen Untersuchungsbereich eines Untersuchungsobjekts in einem ersten Zustand $Z_1$, die zweite Repräsentation $R_2$ repräsentiert den Untersuchungsbereich in einem zweiten Zustand $Z_2$ und die dritte Repräsentation $R_3$ repräsentiert den Untersuchungsbereich in einem dritten Zustand $Z_3$. Die drei Zustände bilden eine Folge von Zuständen: erster Zustand → zweiter Zustand → dritter Zustand.

Die drei Repräsentationen $R_1$, $R_2$ und $R_3$ bilden einen Datensatz innerhalb von Trainingsdaten TD. Die Trainingsdaten TD umfassen eine Vielzahl solcher Datensätze. Der Begriff Vielzahl bedeutet vorzugsweise mehr als 100. Jeder Datensatz umfasst üblicherweise (aber nicht notwendigerweise) drei Repräsentationen des Untersuchungsbereichs zu den drei Zuständen, zu jedem Zustand jeweils eine Repräsentation. Dabei ist der Untersuchungsbereich üblicherweise stets der gleiche und jeder Datensatz umfasst Repräsentationen des Untersuchungsbereich in den drei Zuständen, die ebenfalls üblicherweise für alle Datensätze gleich sind: erster Zustand, zweiter Zustand, dritter Zustand. Lediglich das Untersuchungsobjekt kann variieren. Üblicherweise stammt jeder Datensatz von einem anderen Untersuchungsobjekt. Die in diesem Absatz getätigten Aussagen gelten allgemein, nicht nur in Bezug auf das in Fig. 3 gezeigte Beispiel.

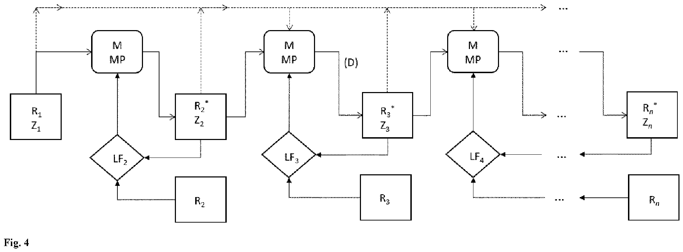

Die Repräsentationen, die zum Trainieren des Modells des maschinellen Lernens verwendet werden, werden in dieser Beschreibung auch als Referenz-Repräsentationen bezeichnet; zur besseren Verständlichkeit/Lesbarkeit wird in der Erläuterung zu Fig. 3 und Fig. 4 auf den Zusatz "Referenz-" verzichtet.

Mittels des Modells des maschinellen Lernens erzeugte (vorhergesagte) Repräsentationen werden in dieser Beschreibung überwiegend durch ein hochgestelltes Stern-Symbol * gekennzeichnet.

In einem ersten Schritt (A) wird die erste Repräsentation $R_1$ einem Modell des maschinellen Lernens M zugeführt. Das Modell des maschinellen Lernens M ist konfiguriert, auf Basis der ersten Repräsentation $R_1$ und auf Basis von Modellparametern MP eine Ausgabe $R_2^*$ zu erzeugen (Schritt (B)). Die Ausgabe $R_2^*$ soll der zweiten Repräsentation $R_2$ möglichst nahekommen; im Idealfall kann die Ausgabe $R_2^*$ nicht von der zweiten Repräsentation R2 unterschieden werden. Mit anderen Worten: bei der Ausgabe $R_2^*$ handelt es sich um eine vorhergesagte zweite Repräsentation. Die Ausgabe $R_2^*$ wird mit der (realen) zweiten Repräsentation $R_2$ verglichen und die Abweichungen werden mit Hilfe einer Fehlerfunktion $LF_2$ quantifiziert. Für jedes Paar von Ausgabe $R_2^*$ und zweite Repräsentation $R_2$ kann ein Fehlerwert $LV_2$ mittels der Fehlerfunktion $LF_2$ berechnet werden.

Beispiele für Fehlerfunktionen, die allgemein (nicht beschränkt auf das Beispiel in Fig. 3) zur Ausführung der vorliegenden Erfindung verwendet werden können, sind L1-Fehlerfunktion (*L1 loss*), L2-Fehlerfunktion (*L2 loss*), Lp-Fehlerfunktion (Lp loss), Strukturähnlichkeitsindexmaß (*structural similarity index measure* (SSIM)), VGG-Fehlerfunktion (VGG loss), Wahrnehmungs-Fehlerfunktion (*perceptual loss*) oder eine Kombination der oben genannten Funktionen oder andere Fehlerfunktionen. Weitere Einzelheiten zu Fehlerfunktionen sind z.B. in der wissenschaftlichen Literatur zu finden (siehe z. B.: R. Mechrez et al.: The Contextual Loss for Image Transformation with Non-Aligned Data, 2018, arxiv:1803.02077v4, H. Zhao et al.: Loss Functions for Image Restoration with Neural Networks, 2018, arxiv:1511.08861v3).

Die Ausgabe $R_2^*$ wird in Schritt (C) erneut dem Modell des maschinellen Lernens M zugeführt. Auch wenn Fig. 3 einen anderen Eindruck erwecken mag: bei dem Modell des maschinellen Lernens M, dem in Schritt (A) die erste

Repräsentation $R_1$ und in Schritt (C) die vorhergesagte zweite Repräsentation $R_2^*$ zugeführt werden, handelt es sich um dasselbe Modell. Das heißt, das Modell des maschinellen Lernens ist nicht nur konfiguriert, auf Basis der ersten Repräsentation eine vorhergesagte zweite Repräsentation zu erzeugen, sondern es ist auch konfiguriert, aus einer (vorhergesagten und/oder realen) zweiten Repräsentation eine Ausgabe $R_3^*$ zu erzeugen, die der dritten Repräsentation $R_3$ möglichst nahekommt. Die Ausgabe $R_3^*$ ist eine vorhergesagte dritte Repräsentation. Die Ausgabe $R_3^*$ wird mit der dritten Repräsentation $R_3$ verglichen. Mit Hilfe einer Fehlerfunktion $LF_3$ kann die Abweichung zwischen der Ausgabe $R_3^*$ und der dritten Repräsentation quantifiziert werden; es kann für jedes Paar einer dritten Repräsentation und einer vorhergesagten dritten Repräsentation ein Fehlerwert $LV_3$ ermittelt werden.

Vorzugsweise wird das Modell des maschinellen Lernens Ende-zu-Ende (engl. *end-to-end*) trainiert. Das bedeutet, dass das Modell des maschinellen Lernens gleichzeitig trainiert wird, eine vorhergesagte zweite Repräsentation auf Basis der ersten Repräsentation und eine vorhergesagte dritte Repräsentation auf Basis der vorhergesagten zweiten Repräsentation zu erzeugen. Dazu wird eine Fehlerfunktion verwendet, die sowohl die Abweichungen zwischen der vorhergesagten zweiten Repräsentation und der zweiten Repräsentation als auch die Abweichungen zwischen der vorhergesagten dritten Repräsentation und der dritten Repräsentation berücksichtigt.

Es ist möglich, die Abweichungen zwischen der zweiten Repräsentation und der vorhergesagten zweiten Repräsentation mit Hilfe der Fehlerfunktion $LF_2$ zu quantifizieren und die Abweichungen zwischen der dritten Repräsentation und der vorhergesagten dritten Repräsentation mit Hilfe der Fehlerfunktion $LF_3$ zu quantifizieren. Eine Fehlerfunktion LF, die beide Abweichungen berücksichtigt, kann beispielsweise die Summe der einzelnen Fehlerfunktionen sein: $LF = LF_2 + LF_3$. Es ist auch möglich die Anteile, die die einzelnen Fehlerfunktionen $LF_2$ und $LF_3$ an der Gesamtfehlerfunktion LF haben, unterschiedlich zu gewichten: $LF = w_2 \cdot LF_2 + w_3 \cdot LF_3$, wobei $w_2$ und $w_3$ Gewichtsfaktoren sind, die beispielsweise Werte zwischen 0 und 1 annehmen können. Dabei kann der Wert Null für einen Gewichtsfaktor beispielsweise dann verwendet werden, wenn in einem Datensatz eine Repräsentation fehlt (mehr dazu ist in der Beschreibung weiter hinten zu finden).

Bei der (späteren) Verwendung des trainierten Modells des maschinellen Lernens zur Vorhersage von neuen Repräsentationen kann es darum gehen, eine dritte Repräsentation auf Basis einer ersten Repräsentation vorherzusagen. Prinzipiell könnte für diesen Zweck ein Modell trainiert werden, die dritte Repräsentation direkt auf Basis der ersten Repräsentation zu erzeugen. Erfindungsgemäß wird das Modell des maschinellen Lernens jedoch trainiert, die dritte Repräsentation nicht in einem Schritt auf Basis der ersten Repräsentation zu erzeugen, sondern in zwei Schritten, wobei in einem ersten Schritt eine zweite Repräsentation vorhergesagt wird, auf deren Basis dann die dritte Repräsentation vorhergesagt wird. Der Vorteil bei dem erfindungsgemäßen Ansatz gegenüber dem genannten "direkten Trainieren" besteht unter anderem darin, dass zusätzliche Trainingsdaten (zweite Repräsentationen, die den Untersuchungsbereich in dem zweiten Zustand repräsentieren), verwendet werden können und damit eine höhere Vorhersagegenauigkeit erreicht werden kann. Ferner lernt das Modell keine Abbildung von einer Repräsentation auf eine andere (oder, wie im Fall von WO2021/052896A1, eine Abbildung von mehreren Repräsentationen auf eine Repräsentation), sondern es lernt das dynamische Verhalten des Untersuchungsbereichs, d.h. die Dynamik, wie die Zustände von einem zum anderen durchlaufen werden. Je mehr Zustände die Trainingsdaten abdecken, desto genauer kann das Modell die Dynamik von einem Zustand zu den nachfolgenden Zuständen lernen. Ferner kann ein so trainiertes Modell verwendet werden, um Repräsentationen zu solchen Zuständen vorherzusagen, für die keine Trainingsdaten vorlagen, indem das Modell mehrfach (iterativ) verwendet wird, um Repräsentationen weiterer nachfolgender Zustände vorherzusagen. Diese Extrapolation auf Zustände, die im Rahmen des Trainings nicht berücksichtigt wurden, ist weiter hinten in der Beschreibung näher beschrieben.

Das in Fig. 3 dargestellte Verfahren ist eine bevorzugte Ausführungsform der vorliegenden Erfindung und umfasst zusammengefasst die Schritte:

- Empfangen einer ersten Repräsentation $R_1$ eines Untersuchungsbereichs, wobei die erste Repräsentation $R_1$ den Untersuchungsbereich in einem ersten Zustand $Z_1$ einer Folge von Zuständen $Z_1$, $Z_2$, $Z_3$ repräsentiert,

- Erzeugen einer vorhergesagten zweiten Repräsentation $R_2^*$ des Untersuchungsbereichs mit Hilfe eines Modells des maschinellen Lernens zumindest anteilig auf Basis der ersten Repräsentation $R_1$, wobei die zweite Repräsentation $R_2^*$ den Untersuchungsbereich in dem zweiten Zustand $Z_2$ repräsentiert, wobei der zweite Zustand $Z_2$ in der Folge von Zuständen unmittelbar auf den ersten Zustand $Z_1$ folgt,

- Erzeugen einer vorhergesagten dritten Repräsentation $R_3^*$ des Untersuchungsbereichs mit Hilfe des Modells des maschinellen Lernens zumindest anteilig auf Basis der vorhergesagten zweiten Repräsentation $R_2^*$, wobei die vorhergesagte dritte Repräsentation $R_3^*$ den Untersuchungsbereich in dem dritten Zustand $Z_3$ repräsentiert, wobei der dritte Zustand $Z_3$ in der Folge von Zuständen unmittelbar auf den zweiten Zustand $Z_2$ folgt,

- Ausgeben und/oder Speichern und/oder Übermitteln der dritten Repräsentation $R_3^*$,

wobei das Modell des maschinellen Lernens auf Basis von Trainingsdaten trainiert wurde, wobei die Trainingsdaten eine Vielzahl von Datensätzen umfasst, wobei jeder Datensatz eine erste Referenz-Repräsentation, eine zweite Referenz-Repräsentation und eine dritte Referenz-Repräsentation umfasst, wobei die erste Referenz-Repräsentation den Untersuchungsbereich in dem ersten Zustand $Z_1$ der Folge von Zuständen repräsentiert, die zweite Referenz-Repräsentation den Untersuchungsbereich in dem zweiten Zustand $Z_2$ der Folge von Zuständen repräsentiert und die dritte Referenz-Repräsentation den Untersuchungsbereich in dem dritten Zustand $Z_3$ der Folge von Zuständen repräsentiert, wobei das Modell des maschinellen Lernens trainiert wurde, zumindest anteilig auf Basis der ersten Referenz-Repräsentation die zweite Referenz-Repräsentation vorherzusagen und zumindest anteilig auf Basis der vorhergesagten zweiten Referenz Repräsentation die dritte Referenz-Repräsentation zu vorherzusagen.

Allgemein kann das erfindungsgemäße Modell des maschinellen Lernens trainiert werden, eine Repräsentation eines Untersuchungsbereichs in einem Zustand $Z_i$ einer Folge von Zuständen $Z_1$ bis $Z_n$ vorherzusagen, wobei $n$ eine ganze Zahl größer als 2 ist und $i$ ein Index ist, der die Zahlen von 2 bis $n$ durchläuft. Dabei kann das Modell des maschinellen Lernens trainiert werden, ausgehend von einer ersten Repräsentation des Untersuchungsbereichs im Zustand $Z_1$ nacheinander eine Folge von vorhergesagten Repräsentationen des Untersuchungsbereichs in den Zuständen $Z_2$ bis $Z_n$ zu erzeugen, wobei jede Repräsentation eines Zustands zumindest anteilig auf Basis der vorhergesagten Repräsentation des unmittelbar vorangegangenen Zustands erzeugt wird.

Dies sei am Beispiel von Fig. 4 näher erläutert. Fig. 4 kann als eine Erweiterung des in Fig. 3 gezeigten Schemas von drei Zuständen auf $n$ Zustände verstanden werden, wobei $n$ eine ganze Zahl größer als zwei ist. Vorzugsweise liegt die Zahl $n$ im Bereich von 3 bis 100.

Fig. 4 zeigt ein Modell des maschinellen Lernens M. Das Modell ist dreimal aufgeführt; es handelt sich aber stets um dasselbe Modell. Das Modell des maschinellen Lernens M wird trainiert, eine Zahl $n$-1 an Repräsentationen vorherzusagen. Im vorliegenden Beispiel ist $n$ eine ganze Zahl größer als 3. Dem Modell wird eine erste Repräsentation $R_1$ als Eingabedaten zugeführt. Die erste Repräsentation $R_1$ repräsentiert einen Untersuchungsbereich eines Untersuchungsobjekts in einem ersten Zustand $Z_1$. Das Modell des maschinellen Lernens M ist konfiguriert, zumindest anteilig auf Basis der ersten Repräsentation $R_1$ und auf Basis von Modellparametern MP eine Ausgabe $R_2^*$ zu erzeugen. Die Ausgabe $R_2^*$ stellt eine vorhergesagte zweite Repräsentation dar, die den Untersuchungsbereich in einem zweiten Zustand $Z_2$ repräsentiert. Das Modell ist ferner konfiguriert, zumindest anteilig auf Basis der Ausgabe $R_2^*$ und Modellparametern MP eine Ausgabe $R_3^*$ zu erzeugen. Die Ausgabe $R_3^*$ stellt eine vorhergesagte dritte Repräsentation dar, die den Untersuchungsbereich in einem dritten Zustand $Z_3$ repräsentiert. Das Modell ist ferner konfiguriert, zumindest anteilig auf Basis der Ausgabe $R_3^*$ und Modellparametern MP eine weitere Ausgabe zu erzeugen, die eine vorgesagte Repräsentation des Untersuchungsbereichs in einem Zustand zeigt, der auf den Zustand $Z_3$ folgt. Dieses Schema wird bis zu einer Ausgabe $R_n^*$ fortgesetzt. Die Ausgabe $R_n^*$ stellt eine vorhergesagte $n$-te Repräsentation dar, die den Untersuchungsbereich in einem $n$-ten Zustand $Z_n$ repräsentiert.

Die Zustände $Z_i$ (mit $i$ = 1 bis $n$) bilden eine Folge von Zuständen ($Z_1 \rightarrow Z_2 \rightarrow Z_3 \rightarrow ... \rightarrow Z_n$).

Das in Fig. 4 dargestellte Verfahren zum Trainieren des Modells des maschinellen Lernens umfasst zusammengefasst die folgenden Schritte:

- Empfangen von Trainingsdaten, wobei die Trainingsdaten eine Vielzahl von Datensätzen einer Vielzahl von Referenz-Untersuchungsobjekten umfassen, wobei die Datensätze Referenz-Repräsentationen eines Untersuchungsbereichs umfassen,

  wobei jeder Datensatz eine oder mehrere Referenz-Repräsentationen einer Zahl $n$ an Referenz-Repräsentationen umfasst,

  wobei jede Referenz-Repräsentation $R_i$ den Untersuchungsbereich in einem Zustand $Z_i$ repräsentiert, wobei $i$ ein Index ist, der die Zahlen von 1 bis $n$ durchläuft,

  wobei die Zustände $Z_1$, ..., $Z_n$ eine Folge von Zuständen bilden,

- Trainieren des Modells des maschinellen Lernens, wobei das Modell trainiert wird, ausgehend von der Referenz-Repräsentation $R_1$ nacheinander vorhergesagte Repräsentationen $R_2^*$ bis $R_n^*$ zu erzeugen, wobei die Erzeugung der vorhergesagten Repräsentation $R_2^*$ zumindest anteilig auf Basis der Referenz-Repräsentation $R_1$ erfolgt und die Erzeugung jeder nachfolgenden vorhergesagten Repräsentation $R_k^*$ zumindest anteilig auf Basis der vorhergesagten Repräsentation $R_{k-1}^*$ erfolgt, wobei $k$ ein Index ist, der die Zahlen von 3 bis $n$ durchläuft, wobei die vorhergesagte Repräsentation $R_k^*$ den Untersuchungsbereich im Zustand $Z_k$ repräsentiert und de vorhergesagte Repräsentation $R_{k-1}^*$ den Untersuchungsbereich im Zustand $Z_{k-1}$ repräsentiert, und der Zustand $Z_{k-l}$ dem Zustand $Z_k$ unmittelbar vorangeht.

Im Fall des in Fig. 4 gezeigten Beispiels liegen, neben der ersten Repräsentation $R_1$, weitere (reale, messtechnisch erzeugte) Repräsentationen $R_j$ (mit $j$ = 2 bis $n$) des Untersuchungsbereichs in den Zuständen $Z_j$ (mit $j$ = 2 bis $n$) vor, die als Zieldaten (*ground truth*) zum Trainieren des Modells des maschinellen Lernens verwendet werden können. So kann die Abweichung zwischen einer Ausgabe $R_j^*$ (die eine vorgesagte $j$-te Repräsentation des Untersuchungsbereichs im Zustand $Z_j$ darstellt) und der Repräsentation $R_j$ mit einer Fehlerfunktion $LF_j$ quantifiziert werden: eine Fehlerfunktion $LF_2$ kann die Abweichung zwischen der Repräsentation $R_2$ und der vorhergesagten Repräsentation $R_2^*$ quantifizieren, eine Fehlerfunktion $LF_3$ kann die Abweichung zwischen der Repräsentation $R_3$ und der vorhergesagten Repräsentation $R_3^*$ quantifizieren, und so fort.

In einem Ende-zu-Ende-Training kann eine Gesamtfehlerfunktion LF verwendet werden, die alle einzelnen Abweichungen berücksichtigt; dabei kann es sich beispielsweise um die Summe der einzelnen Abweichungen handeln:

$$LF = LF_2 + LF_3 + \ldots + LF_n = \sum_{j=2}^{n} LF_j$$

Es kann sich aber auch um eine gewichtete Summe handeln:

$$LF = w_2 \cdot LF_2 + w_3 \cdot LF_3 + \ldots + w_n \cdot LF_n = \sum_{j=2}^{n} w_j \cdot LF_j$$

wobei die Gewichtsfaktoren $w_j$ beispielsweise Werte von 0 bis 1 annehmen können.

Die Gewichtung hat den Vorteil, dass bei der Erzeugung einer vorhergesagten Repräsentation $R_n^*$, die den Untersuchungsbereich in einem Zustand $Z_n$ repräsentiert, einer oder mehreren vorhergesagten Repräsentationen, die einen Zustand repräsentieren, der vor dem Zustand $Z_n$ liegt/liegen, mehr oder weniger Gewicht beigemessen wird, als anderen Repräsentationen. So ist es beispielweise möglich, dass die Gewichtsfaktoren $w_i$ in der Reihe $w_2$, $w_3$, ..., $w_n$ zunehmen und damit Repräsentationen, deren Zustände näher an dem Zustand $Z_n$ liegt, höher gewichtet werden. Dabei kann die Zunahme logarithmisch, linear, quadratisch, kubisch oder exponentiell sein oder eine andere Zunahme sein. Es ist auch denkbar, späteren Zuständen weniger Gewicht beizumessen, indem die Gewichtsfaktoren in der Reihe $w_2$, $w_3$, ..., $w_n$ abnehmen und damit Repräsentationen, deren Zustände näher an dem initialen Zustand $Z_1$ liegen mehr Gewicht beimessen. Auch eine solche Abnahme kann logarithmisch, linear, quadratisch, kubisch oder exponentiell sein oder eine andere Abnahme sein.

Es ist auch denkbar, dass die Trainingsdaten unvollständige Datensätze umfassen. Das bedeutet, dass einige Datensätze von Untersuchungsobjekten nicht alle Referenz-Repräsentationen $R_1$ bis $R_n$ umfassen. Wie weiter hinten in der Beschreibung erläutert, können auch unvollständige Datensätze zum Trainieren des Modells des maschinellen Lernens verwendet werden. Falls eine Referenz-Repräsentation $R_p$ fehlt, kann der Gewichtsfaktor $w_p$, der die Fehlerfunktion zu der Referenz-Repräsentation $R_p$ und der vorhergesagten Referenz-Repräsentation $R_p^*$ gewichten soll, auf Null gesetzt werden, wobei $p$ eine ganze Zahl ist, die die Werte 2 bis $n$ annehmen kann.

Es ist auch denkbar, dass bei dem Training zufällige Zahlen $1 \leq j < k \leq n$ und die damit verbundenen Teilfolgen von Zuständen $Z_j$, $Z_{j+1}$, ..., $Z_{k-1}$, $Z_k$ betrachtet werden. Das oben beschriebene Lernproblem kann dann auf diesen (optional von Zeit zu Zeit variierenden) Teilfolgen gelöst werden. Zum Beispiel ist denkbar, dass ein zufälliger initialer Zustand $Z_j$ ($1 \leq j \leq n$-2) bestimmt wird und das Modell auf dessen Basis stets die Repräsentationen der nachfolgenden zwei Zustände $Z_{j+1}$, $Z_{j+2}$ synthetisieren soll.

In Fig. 4 ist auch schematisch dargestellt, dass zur Erzeugung einer vorhergesagten Repräsentation $R_j^*$, die den Untersuchungsbereich in einem Zustand $Z_j$ repräsentiert, neben der vorhergesagten Repräsentation $R_{j-1}^*$, die den Untersuchungsbereich in einem Zustand $Z_{j-1}$ repräsentiert, auch weitere (vorhergesagte) Repräsentation $R_{j-2}^*$ und/oder $R_{j-3}^*$ und/oder ... bis $R_1$, die den Untersuchungsbereich in weiteren vorangegangenen Zuständen $Z_{j-2}$, $Z_{j-3}$ ... bis $Z_1$ repräsentieren, verwendet werden können, wobei $j$ eine ganze Zahl ist, die die Werte 2 bis $n$ annehmen kann. Dies wird durch die gestrichelten Pfeile zum Ausdruck gebracht. So kann zur Vorhersage der dritten Repräsentation neben der vorhergesagten zweiten Repräsentation auch die erste Repräsentation dem Modell des maschinellen Lernens zugeführt werden. Zur Vorhersage einer vierten Repräsentation kann neben der vorhergesagten dritten Repräsentation auch die erste Repräsentation und/oder die vorhergesagte zweite Repräsentation dem Modell des maschinellen Lernens zugeführt werden.

Es können zur Vorhersage von Repräsentationen auch weitere Informationen in das Modell des maschinellen Lernens einfließen, wie beispielsweise Informationen über den Zustand, in dem sich der Untersuchungsbereich befindet, dessen Repräsentation zur Vorhersage einer Repräsentation in einem nachfolgenden Zustand verwendet wird. Mit anderen Worten: neben der ersten Repräsentation können zur Vorhersage der zweiten Repräsentation auch Informationen zu dem Zustand, in dem sich der Untersuchungsbereich, der durch die erste Repräsentation repräsentiert wird, befindet, verwendet werden. Auch die vorhergesagte dritte Repräsentation kann auf Basis der vorhergesagten zweiten Repräsentationen und Informationen zu dem zweiten Zustand erzeugt werden. Wird eine Information über den jeweils vorliegenden Zustand einer Repräsentation mitgegeben, so "weiß" das Modell des maschinellen Lernens, an welcher Stelle der Folge von Zuständen es sich jeweils befindet.

Ein Zustand kann beispielsweise durch eine Zahl oder einen Vektor repräsentiert werden. Die Zustände können zum Beispiel durchnummeriert werden, so dass der erste Zustand durch die Zahl 1 repräsentiert wird, der zweite Zustand durch die Zahl 2 und so fort.

Anstelle oder in Ergänzung zu einer Information zum jeweils vorliegenden Zustand kann auch eine Zeit, die dem jeweiligen Zustand zugeordnet werden kann, in die Vorhersage einer Repräsentation einfließen. So kann dem ersten Zustand in der Folge der Zustände z.B. der Zeitpunkt $t=0$ zugeordnet werden. Die Zeit $t=0$ kann zusammen mit der ersten Repräsentation in das Modell des maschinellen Lernens eingegeben werden, um die zweite Repräsentation vorherzusagen. Dem zweiten Zustand kann eine Zahl an Sekunden oder Minuten oder eine andere Einheit einer Zeitdifferenz zugeordnet werden, die angibt, wieviel Zeit zwischen dem ersten Zustand und dem zweiten Zustand vergangen ist. Diese Zeitdifferenz kann zusammen mit der vorhergesagten zweiten Repräsentation (und optional der ersten Repräsentation) dem Modell des maschinellen Lernens zugeführt werden, um die dritte Repräsentation vorherzusagen. Dem dritten Zustand kann ebenso eine Zeitdifferenz zugeordnet werden, die angibt, wieviel Zeit zwischen dem ersten Zustand und dem dritten Zustand vergangen ist. Diese Zeitdifferenz kann zusammen mit der vorhergesagten dritten Repräsentation (und optional der ersten Repräsentation und/oder der vorhergesagten zweiten Repräsentation) dem Modell des maschinellen Lernens zugeführt werden, um die dritte Repräsentation vorherzusage. Und so fort.

Das erfindungsgemäße Modell des maschinellen Lernens kann auch als eine Transformation verstanden werden, die auf eine Repräsentation eines Untersuchungsbereichs in einem Zustand einer Folge von Zuständen angewendet werden kann, um eine Repräsentation des Untersuchungsbereichs in einem nachfolgenden Zustand der Folge von Zuständen vorherzusagen. Die Transformation kann einfach (einmal) angewendet werden, um eine Repräsentation des Untersuchungsbereichs in dem unmittelbar nachfolgenden Zustand vorherzusagen, oder mehrfach (mehrmals, iterativ) angewendet werden, um eine Repräsentation des Untersuchungsbereichs in einem in der Folge der Zustände weiter hinten liegenden Zustand vorherzusagen.

Liegt eine Folge von $n$ Zuständen $Z_1$ bis $Z_n$ vor, und liegt zu jedem Zustand $Z_i$ eine Repräsentation $R_i$ vor, die den Untersuchungsbereich in dem Zustand $Z_i$ repräsentiert, so kann das Modell M des maschinellen Lernens ausgehend von der Repräsentation $R_1$ $q$-fach angewendet werden, um eine vorhergesagte Repräsentation $R_{1+q}^*$ zu erzeugen, die den Untersuchungsbereich in dem Zustand $Z_{1+q}$ repräsentiert, wobei $q$ die Werte 1 bis $n$-1 annehmen kann:

$$\mathbf{M}^q(R_1) = R_{1+q}^*$$

$$q = 1: \mathbf{M}(R_1) = R_2^*$$

$$q = 2: \mathbf{M}(R_2^*) = \mathbf{M}(\mathbf{M}(R_1)) = \mathbf{M}^2(R_1) = R_3^*$$

$$q = 3: \mathbf{M}(R_3^*) = \mathbf{M}(\mathbf{M}(R_2^*)) = \mathbf{M}(\mathbf{M}(\mathbf{M}(R_1))) = \mathbf{M}^3(R_1) = R_4^*$$

$$q = n\text{-}1: \mathbf{M}(R_{n-1}^*) = \mathbf{M}(\mathbf{M}(R_{n-2}^*)) = \ldots = \mathbf{M}^{n-1}(R_1) = R_n^*$$

Eine Fehlerfunktion, die alle Abweichungen zwischen vorhergesagten Repräsentationen $R_q^*$ und realen (messtechnisch erzeugten) Repräsentationen $R_q$ quantifiziert, kann beispielsweise durch die folgende Formel ausgedrückt werden:

$$LV = w_2 \cdot \mathbf{d}(\mathbf{M}(R_1), R_2) + w_3 \cdot \mathbf{d}(\mathbf{M}^2(R_1), R_3) + \ldots + w_n \cdot \mathbf{d}(\mathbf{M}^{n-1}(R_1), R_n)$$

Dabei ist LV der Fehlerwert, der sich für einen Datensatz umfassend die Referenz-Repräsentationen $R_1$, $R_2$, ..., $R_n$ ergibt. $\mathbf{d}$ ist eine Fehlerfunktion, die die Abweichungen zwischen einer vorhergesagten Repräsentation $\mathbf{M}(R_{q-1})$ und der Referenz-Repräsentation $R_q$ quantifiziert. Wie bereits weiter vorne in der Beschreibung beschrieben, kann es sich dabei beispielsweise um eine der folgenden Fehlerfunktionen handeln: L1 loss, L2 loss, Lp loss, Strukturähnlichkeitsindexmaß (SSIM), *VGG loss, perceptual loss* oder eine Kombination davon.

$w_2$, $w_3$, ..., $w_n$ sind Gewichtsfaktoren, die ebenfalls weiter vorne in der Beschreibung bereits beschrieben wurden.

$n$ gibt die Zahl der Zustände an.

Es ist auch denkbar, dass der Fehlerwert die maximale Abweichung ist, die für einen Datensatz berechnet wird:

$$LV = \max(w_2 \cdot \mathbf{d}(\mathbf{M}(R_1), R_2), w_3 \cdot \mathbf{d}(\mathbf{M}^2(R_1), R_3), \ldots, w_n \cdot \mathbf{d}(\mathbf{M}^{n-1}(R_1), R_n))$$

wobei auch in dieser Formel durch die Gewichtsfaktoren den einzelnen Abweichungen ein unterschiedliches Gewicht beigemessen werden kann.

Wie bereits weiter vorne in der Beschreibung angedeutet, sei angemerkt, dass es zum Trainieren des Modells des maschinellen Lernens nicht erforderlich ist, dass jeder Datensatz der Trainingsdaten Repräsentationen von allen Zuständen, die das Modell lernen soll, beinhaltet. Dies sei an einem Beispiel erläutert. Es sei angenommen, dass das Modell des maschinellen Lernens trainiert werden soll, Repräsentationen eines Untersuchungsbereichs in den Zuständen einer Folge von 6 Zuständen $Z_1$ bis $Z_6$ vorherzusagen. Es sei angenommen, dass zum Trainieren des Modells des maschinellen Lernens Trainingsdaten ausreichend sind, die 10 Datensätze von 10 Untersuchungsobjekten umfassen. Jeder Datensatz umfasst Repräsentationen des Untersuchungsbereichs in verschiedenen Zuständen, z.B.:

Datensatz 1: $R_1$, $R_3$, $R_4$, $R_5$, $R_6$
Datensatz 2: $R_1$, $R_2$, $R_4$, $R_6$
Datensatz 3: $R_1$, $R_2$, $R_3$, $R_4$, $R_5$
Datensatz 4: $R_1$, $R_2$, $R_3$, $R_3$, $R_6$
Datensatz 5: $R_2$, $R_3$, $R_5$, $R_6$
Datensatz 6: $R_2$, $R_3$, $R_4$, $R_5$, $R_6$
Datensatz 7: $R_2$, $R_3$, $R_5$, $R_6$
Datensatz 8: $R_3$, $R_5$, $R_6$
Datensatz 9: $R_3$, $R_4$, $R_5$, $R_6$
Datensatz 10: $R_3$, $R_4$, $R_6$

In dem Beispiel gibt es keinen einzigen "vollständigen" Datensatz, also keinen Datensatz, der alle möglichen Repräsentationen $R_1$, $R_2$, $R_5$, $R_4$, $R_5$ und $R_6$ umfasst. Dennoch ist es möglich, das Modell des maschinellen Lernens auf Basis von solchen Trainingsdaten zu trainieren, eine Repräsentation eines jeden Zustands vorherzusagen. Dies ist ein Vorteil der vorliegenden Erfindung gegenüber dem oben beschriebenen "direkten Trainieren".

Ist das Modell des maschinellen Lernens trainiert, können dem Modell neue (d.h. nicht im Training verwendete) Repräsentationen eines Untersuchungsbereichs in einem Zustand einer Folge von Zuständen zugeführt werden, und das Modell kann eine oder mehrere Repräsentationen des Untersuchungsbereichs in einem nachfolgenden Zustand oder in mehreren nachfolgenden Zuständen der Folge von Zuständen vorhersagen (erzeugen).

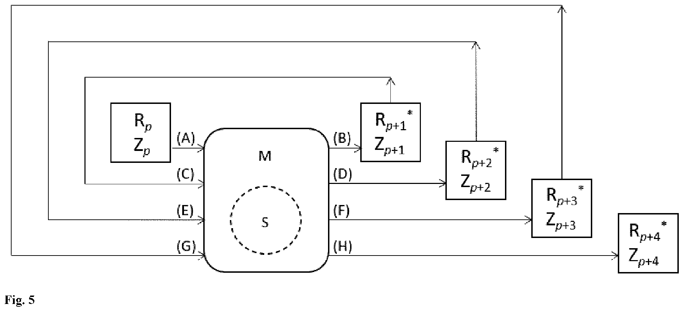

Dies ist beispielhaft und schematisch in Fig. 5 dargestellt. In dem in Fig. 5 dargestellten Beispiel werden ausgehend von einer Repräsentation $R_p$, die den Untersuchungsbereich in dem Zustand $Z_p$ repräsentiert, eine Folge von Repräsentationen $R_{p+1}^*$, $R_{p+2}^*$, $R_{p+3}^*$ und $R_{p+4}^*$ erzeugt, die den Untersuchungsbereich in den Zuständen $Z_{p+1}$, $Z_{p+2}$, $Z_{p+3}$ und $Z_{p+4}$ repräsentieren. Die Zustände $Z_{p+1}$, $Z_{p+2}$, $Z_{p+3}$ und $Z_{p+4}$ bilden eine Folge von Zuständen: $Z_{p+1} \rightarrow Z_{p+2} \rightarrow Z_{p+3} \rightarrow Z_{p+4}$.

In Schritt (A) wird dem Modell des maschinellen Lernens M die Repräsentation $R_p$ zugeführt. Neben der Repräsentation $R_p$ können dem Modell des maschinellen Lernens M auch Informationen zu dem Zustand $Z_p$ und/oder weitere/andere Informationen zugeführt werden, wie in dieser Beschreibung beschrieben.

Das Modell des maschinellen Lernens M erzeugt in Schritt (B) auf Basis der zugeführten Eingabedaten die vorhergesagte Repräsentation $R_{p+1}^*$, die den Untersuchungsbereich in dem Zustand $Z_{p+1}$ repräsentiert.

In Schritt (C) wird dem Modell des maschinellen Lernens M die zuvor erzeugte Repräsentation $R_{p+1}^*$ zugeführt. Neben der Repräsentation $R_{p+1}^*$ können dem Modell des maschinellen Lernens M auch Informationen zu dem Zustand $Z_{p+1}$ und/oder weitere Informationen zugeführt werden. Ferner können dem Modell des maschinellen Lernens auch die Repräsentation $R_p$ und/oder Informationen zu dem Zustand $Z_p$ zugeführt werden.

Vorzugsweise umfasst das Modell des maschinellen Lernens M einen Speicher S, der Eingabedaten (und vorzugsweise auch Ausgabedaten) speichert, so dass einmal eingegebene Eingabedaten und/oder erzeugte Ausgabedaten nicht erneut empfangen und/oder eingegeben werden müssen, sondern dem Modell des maschinellen Lernens bereits zur Verfügung stehen. Dies gilt nicht nur für die in diesem Beispiel beschriebene Nutzung des trainierten Modells des maschinellen Lernens zur Vorhersage, sondern auch für das Trainieren des erfindungsgemäßen Modells des maschinellen Lernens.

Das Modell des maschinellen Lernens M erzeugt in Schritt (D) auf Basis der zugeführten Eingabedaten die vorhergesagte Repräsentation $R_{p+2}^*$, die den Untersuchungsbereich in dem Zustand $Z_{p+2}$ repräsentiert.

In Schritt (E) wird dem Modell des maschinellen Lernens M die zuvor erzeugte Repräsentation $R_{p+2}^*$ zugeführt. Neben der vorhergesagten Repräsentation $R_{p+2}^*$ können dem Modell des maschinellen Lernens M auch Informationen zu dem Zustand $Z_{p+2}$ und/oder weitere Informationen zugeführt werden. Ferner können dem Modell des maschinellen Lernens auch die Repräsentation $R_p$ und/oder die vorhergesagte Repräsentation $R_{p+1}^*$ und/oder Informationen zu dem

Zustand $Z_p$ und/oder dem Zustand $Z_{p+1}$ zugeführt werden.

Das Modell des maschinellen Lernens M erzeugt in Schritt (F) auf Basis der zugeführten Eingabedaten die vorhergesagte Repräsentation $R_{p+3}^*$, die den Untersuchungsbereich in dem Zustand $Z_{p+3}$ repräsentiert.

In Schritt (G) wird dem Modell des maschinellen Lernens M die zuvor erzeugte Repräsentation $R_{p+3}^*$ zugeführt. Neben der vorhergesagten Repräsentation $R_{p+3}^*$ können dem Modell des maschinellen Lernens M auch Informationen zu dem Zustand $Z_{p+3}$ und/oder weitere Informationen zugeführt werden. Ferner können dem Modell des maschinellen Lernens auch die Repräsentation $R_p$ und/oder die vorhergesagte Repräsentation $R_{p+1}^*$ und/oder die vorhergesagte Repräsentation $R_{p+2}^*$ und/oder Informationen zu dem Zustand $Z_p$ und/oder dem Zustand $Z_{p+1}$ und/oder dem Zustand $Z_{p+2}$ zugeführt werden.

Das Modell des maschinellen Lernens M erzeugt in Schritt (H) auf Basis der zugeführten Eingabedaten die vorhergesagte Repräsentation $R_{p+4}^*$, die den Untersuchungsbereich in dem Zustand $Z_{p+4}$ repräsentiert.

Die erzeugten Repräsentationen $R_{p+1}^*$, $R_{p+2}^*$, $R_{p+3}^*$ und/oder $R_{p+4}^*$ können ausgegeben (z.B. auf einem Monitor angezeigt und/oder mit einem Drucker ausgedruckt) und/oder in einem Datenspeicher gespeichert und/oder an ein (separates) Computersystem übermittelt werden.

Wurde das erfindungsgemäße Modell des maschinellen Lernens trainiert, ausgehend von einer ersten Repräsentation $R_1$, die den Untersuchungsbereich in einem ersten Zustand $Z_1$ repräsentiert, eine Folge von vorhergesagten Repräsentationen $R_2^*$, ..., $R_n^*$ zu erzeugen, wobei jede Repräsentation $R_j^*$ den Untersuchungsbereich in einem Zustand $Z_j$ repräsentiert, wobei $j$ ein Index ist, der die Zahlen von 2 bis n durchläuft, dann kann einem solchen Modell eine neue Repräsentation $R_p$ zugeführt werden, die den Untersuchungsbereich im Zustand $Z_p$ repräsentiert, und das Modell des maschinellen Lernens kann die vorhergesagten Repräsentationen $R_{p+1}^*$, $R_{p+2}^*$, ... , $R_n^*$ erzeugen, wobei $p$ eine Zahl ist, die die Werte 2 bis $n$ annehmen kann.

Das bedeutet, dass dem trainierten Modell nicht zwangsläufig eine neue Repräsentation $R_1$, die den Untersuchungsbereich in dem ersten Zustand $Z_1$ repräsentiert, zugeführt werden muss, und das trainierte Modell des maschinellen Lernens ist auch nicht darauf beschränkt, nur die Repräsentation $R_n^*$, die den Untersuchungsbereich in dem letzten Zustand $Z_n$ repräsentiert, vorherzusagen. Stattdessen kann man in der Folge von Zuständen $Z_1$ bis $Z_n$ an jeder Stelle "einsteigen" und auf Basis der dem Modell zugeführten Repräsentation eine Repräsentation jedes anderen Zustands vorhersagen.

Es ist sogar möglich, Repräsentationen zu erzeugen, die Zustände repräsentieren, die gar nicht Gegenstand des Trainings gewesen sind. Anstatt also bei der Repräsentation $R_n^*$ zu stoppen, können auch die Repräsentationen $R_{n+1}^*$, $R_{n+2}^*$ und so weiter vorhergesagt werden. Das trainierte Modell des maschinellen Lernens kann also verwendet werden, um die gelernte Dynamik fortzusetzen und Repräsentationen von Zuständen zu berechnen, die messtechnisch niemals erzeugt worden sind. Insofern kann das trainierte Modell des maschinellen Lernens zur Extrapolation auf neue Zustände verwendet werden.

Ferner sind die Vorhersagen nicht auf nachfolgende Zustände beschränkt. Es ist auch möglich Repräsentationen des Untersuchungsbereichs in einem vorangegangenen Zustand der Folge von Zuständen vorherzusagen. Zum einen kann - wie weiter vorne in der Beschreibung bereits beschrieben - das Modell des maschinellen Lernens grundsätzlich in beide Richtungen trainiert werden: in Richtung nachfolgender Zustände und in Richtung vorangegangener Zustände. Zum anderen führt das Modell des maschinellen Lernens an einer eingegebenen Repräsentation eine Transformation aus, die sich prinzipiell auch umkehren lässt. Aus der Analyse der mathematischen Funktionen des Modells, die eine Eingabe-Repräsentation in eine Ausgabe-Repräsentation transformieren, lassen sich Umkehrfunktionen ermitteln, die den Prozess umkehren und die vorherige Ausgabe-Repräsentation wieder in die vorherige Eingabe-Repräsentation zurückverwandelt. Die Umkehrfunktionen können dann verwendet werden, Repräsentationen vorangegangener Zustände vorherzusagen, auch wenn das Modell trainiert worden ist, Repräsentationen nachfolgender Zustände vorherzusagen und umgekehrt.

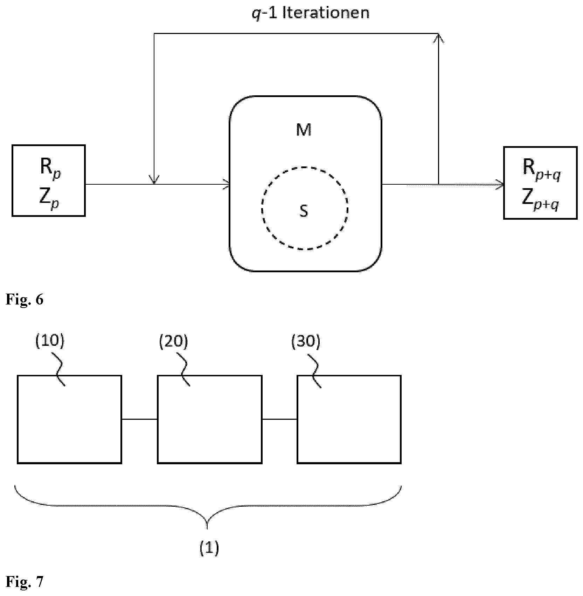

Fig. 6 zeigt eine Erweiterung des in Fig. 5 gezeigten Schemas. Während in Fig. 5 auf Basis einer Repräsentation $R_p$, die den Untersuchungsbereich in dem Zustand $Z_p$ repräsentiert, nacheinander die vorhergesagten Repräsentationen $R_{p+1}^*$, $R_{p+2}^*$, $R_{p+3}^*$ und $R_{p+4}^*$ erzeugt werden, die den Untersuchungsbereich in den Zuständen $Z_{p+1}$, $Z_{p+2}$, $Z_{p+3}$ und $Z_{p+4}$ repräsentieren, ist in Fig. 6 gezeigt, wie auf Basis der Repräsentation $R_p$, die den Untersuchungsbereich in dem Zustand $Z_p$ repräsentiert, nacheinander die vorhergesagten Repräsentationen $R_{p+1}^*$ bis $R_{p+q}^*$ erzeugt werden, wobei $q$ eine ganze Zahl größer als 1 ist. In Fig. 6 kommt der iterative Charakter des Modells des maschinellen Lernens M besonders deutlich zum Ausdruck. Das Modell des maschinellen Lernens M wird, ausgehend von der Repräsentation $R_p$ $q$-mal angewandt, wobei $(q-1)$-mal die Ausgabedaten wieder zurück in das Modell des maschinellen geschleust werden.

Es sei darauf hingewiesen, dass das (trainierte oder untrainierte) Modell des maschinellen Lernens nicht auf eine komplette radiologische Aufnahme (z.B. eine MRT-Aufnahme oder einen CT-Scan oder dergleichen) angewendet werden muss. Es ist möglich, das Modell des maschinellen Lernens nur auf einen Teil einer radiologischen Aufnahme anzuwenden. Es ist beispielsweise möglich, eine radiologische Aufnahme zunächst zu segmentieren, um einen Bereich von Interesse (engl. *region of interest*) zu identifizieren/selektieren. Das Modell kann dann beispielsweise ausschließlich auf den Bereich von Interesse angewandt werden.

Ferner kann die Anwendung des Modells des maschinellen Lernens einen oder mehrere Vorverarbeitungs- und/oder Nachverarbeitungsschritte umfassen. Es Beispiel denkbar, eine empfangene Repräsentation des Untersuchungsbereichs zunächst einer oder mehreren Transformationen zu unterziehen, wie beispielsweise einer Bewegungskorrektur, einer Farbraumumwandlung, einer Normalisierung, einer Segmentierung und/oder dergleichen. In einem weiteren Schritt kann die transformierte Repräsentation dem Modell des maschinellen Lernens zugeführt werden, das dann eine Reihe von Iterationen (Zyklen) durchläuft (wie in Fig. 6 schematisch dargestellt), um ausgehend von der transformierten Repräsentation eine Reihe von weiteren (nachfolgenden) Repräsentationen des Untersuchungsbereich in einer Reihe von weiteren (nachfolgenden) Zuständen zu erzeugen.

Bei dem erfindungsgemäßen Modell des maschinellen Lernens kann es sich beispielsweise um ein künstliches neuronales Netzwerk handeln, oder es kann ein solches umfassen.

Ein künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen der Repräsentationen. Üblicherweise gibt es ein Eingangsneuron für jedes Pixel oder Voxel einer Repräsentation, wenn es sich bei der Repräsentation um eine Ortsraumdarstellung in Form einer Rastergrafik handelt. Es können zusätzliche Eingangsneuronen für zusätzliche Eingangswerte (z.B. Informationen zum Untersuchungsbereich, zum Untersuchungsobjekt, zu Bedingungen, die bei der Erzeugung der Repräsentation herrschten, Informationen zu dem Zustand, den die Repräsentation repräsentiert, und/oder Informationen zu dem Zeitpunkt oder der Zeitspanne zu/in der die Repräsentation erzeugt worden ist) vorhanden sein.

Die Ausgangsneuronen können dazu dienen, eine vorhergesagte Repräsentation, die den Untersuchungsbereich in einem nachfolgenden Zustand repräsentiert, auszugeben.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN) oder es umfasst ein solches.

Ein CNN besteht üblicherweise im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Das Trainieren des neuronalen Netzes kann beispielsweise mittels eines Backpropagation-Verfahrens durchgeführt werden. Dabei wird für das Netz eine möglichst zuverlässige Vorhersage der Dynamik des Untersuchungsbereichs von einem Zustand über mindestens einen Zwischenzustand zu einem Endzustand. Die Qualität der Vorhersage wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich der Dynamik der Zustandsänderungen, die verwendet werden können, um, auf Basis einer ersten Repräsentation, die den Untersuchungsbereich in einem ersten Zustand repräsentiert, eine oder mehrere Repräsentationen, die den Untersuchungsbereich in einem oder mehreren nachfolgenden Zuständen repräsentiert, vorherzusagen.

Eine Kreuzvalidierungsmethode kann verwendet werden, um die Daten in Trainings- und Validierungsdatensätze aufzuteilen. Der Trainingsdatensatz wird beim Backpropagation-Training der Netzwerkgewichte verwendet. Der Validierungsdatensatz wird verwendet, um zu überprüfen, mit welcher Vorhersagegenauigkeit sich das trainierte Netzwerk auf unbekannte Daten anwenden lässt.

Das künstliche neuronale Netzwerk kann eine Autoencoder-Architektur aufweisen; z.B. kann das künstliche neuronale Netzwerk eine Architektur wie das U-Net aufweisen (siehe z.B. O. Ronneberger et al.: U-net: Convolutional networks for biomedical image segmentation, International Conference on Medical image computing and computer-assisted intervention, Seiten 234-241, Springer, 2015, https://doi.org/10.1007/978-3-319-24574-4_28).

Das künstliche neuronale Netzwerk kann ein *Generative Adversarial Network* (GAN) sein (siehe z.B. M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887).

Das künstliche neuronale Netzwerk kann ein rekurrentes neuronales Netzwerk sein oder ein solches umfassen. Als rekurrente bzw. rückgekoppelte neuronale Netzwerke bezeichnet man neuronale Netzwerke, die sich im Gegensatz zu den Feedforward-Netzen durch Verbindungen von Neuronen einer Schicht zu Neuronen derselben oder einer vorangegangenen Schicht auszeichnen. Das künstliche neuronale Netzwerk kann beispielsweise ein *Long short-term memory* (LSTM) umfassen (siehe z.B. Y. Gao et al.: Fully convolutional structured LSTM networks for joint 4D medical image segmentation, DOI: 10.1109/ISBI.2018.8363764).

Das künstliche neuronale Netz kann ein Transfomer-Netzwerk sein (siehe z.B. D. Karimi et al.: Convolution-Free Medical Image Segmentation using Transformers, arXiv:2102.13645 [eess.IV]).

Fig. 7 zeigt beispielhaft und schematisch ein erfindungsgemäßes Computersystem.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Das in Fig. 7 gezeigte Computersystem (1) umfasst eine Eingabeeinheit (10), eine Steuer- und Recheneinheit (20) und eine Ausgabeeinheit (30).

Die Steuer- und Recheneinheit (20) dient der Steuerung des Computersystems (1), der Koordinierung der Datenflüsse zwischen den Einheiten des Computersystems (1) und der Durchführung von Berechnungen.

Die Steuer- und Recheneinheit (20) ist konfiguriert

- über die Eingabeeinheit (10) eine Repräsentation zu empfangen, die einen Untersuchungsbereich in einem ersten Zustand $Z_1$ einer Folge von Zuständen $Z_1$ bis $Z_n$ repräsentiert, wobei $n$ eine ganze Zahl ist, die größer als zwei ist,
- die Repräsentation einem trainierten Modell des maschinellen Lernens zuzuführen, wobei das Modell des maschinellen Lernens auf Basis von Trainingsdaten trainiert worden ist, ausgehend von einer ersten Referenz-Repräsentation $R_1$ eine Zahl $n$-1 von Referenz-Repräsentationen $R_2{}^*$ bis $R_n$ vorherzusagen, wobei die erste Referenz-Repräsentation $R_1$ den Untersuchungsbereich im Zustand $Z_1$ repräsentiert und jede vorhergesagte Referenz-Repräsentation $R_j{}^*$ den Untersuchungsbereich im Zustand $Z_j$ repräsentiert, wobei $j$ ein Index ist, der die Zahlen von 2 bis $n$ durchläuft, wobei die Erzeugung der vorhersagten Referenz-Repräsentation $R_2{}^*$ zumindest anteilig auf Basis der Referenz-Repräsentation $R_1$ erfolgt und die Erzeugung jeder weiteren vorhergesagten Referenz-Repräsentation $R_k{}^*$ zumindest anteilig auf Basis der vorhergesagten Referenz-Repräsentation $R_{k-1}{}^*$ erfolgt, wobei $k$ ein Index ist, der die Zahlen von 3 bis $n$ durchläuft,
- von dem Modell des maschinellen Lernens eine oder mehrere vorhergesagte Repräsentationen des Untersuchungsbereichs zu empfangen, wobei jede der einen oder der mehreren vorhergesagten Repräsentationen den Untersuchungsbereich in einem Zustand repräsentiert, der dem ersten Zustand $Z_1$ nachfolgt,
- die Ausgabeeinheit (30) zu veranlassen die eine oder die mehreren vorhergesagten Repräsentationen auszugeben und/oder zu speichern und/oder an ein separates Computersystem zu übermitteln.

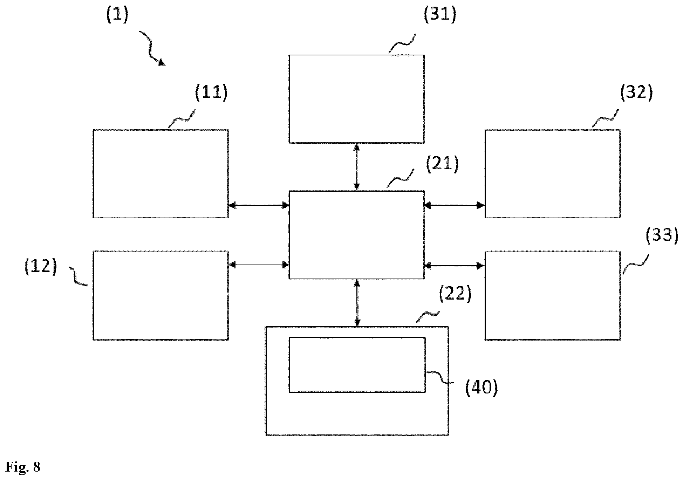

Fig. 8 zeigt beispielhaft und schematisch eine weitere Ausführungsform des erfindungsgemäßen Computersystems.

Das Computersystem (1) umfasst eine Verarbeitungseinheit (21), die mit einem Speicher (22) verbunden ist. Die Verarbeitungseinheit (21) und der Speicher (22) bilden eine Steuer- und Berechnungseinheit, wie sie in Fig. 7 gezeigt ist.

Die Verarbeitungseinheit (21) (engl.: *processing unit*) kann einen oder mehrere Prozessoren allein oder in Kombination mit einem oder mehreren Speichern umfassen. Bei der Verarbeitungseinheit (21) kann es sich um gewöhnliche Computerhardware handeln, die in der Lage ist, Informationen wie z.B. digitale Bildaufnahmen, Computerprogramme und/oder andere digitale Informationen zu verarbeiten. Die Verarbeitungseinheit (21) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können. Die Verarbeitungseinheit (21) kann konfiguriert sein, Computerprogramme auszuführen, die in einem Arbeitsspeicher der Verarbeitungseinheit (21) oder im Speicher (22) desselben oder eines anderen Computersystems gespeichert sein können.

Der Speicher (22) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (22) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (22) kann die Verarbeitungseinheit (21) auch mit einer oder mehreren Schnittstellen (11, 12, 31, 32, 33) verbunden sein, um Informationen anzuzeigen, zu übertragen und/oder zu empfangen. Die Schnittstellen können eine oder mehrere Kommunikationsschnittstellen (32, 33) und/oder eine oder mehrere Benutzerschnittstellen (11, 12, 31) umfassen. Die eine oder mehrere Kommunikationsschnittstellen können so konfiguriert sein, dass sie Informationen senden und/oder empfangen, z.B. zu und/oder von einer MRT-Scanner, einem CT-Scanner, einer Ultraschallkamera, anderen Computersystemen, Netzwerken, Datenspeichern oder dergleichen. Die eine oder mehrere Kommunikationsschnittstellen können so konfiguriert sein, dass sie Informationen über physische (verdrahtete) und/oder drahtlose Kommunikationsverbindungen übertragen und/oder empfangen. Die eine oder die mehreren Kommunikationsschnittstellen können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen eine oder mehrere Nahbereichskommunikationsschnittstellen umfassen, die so konfiguriert sind, dass sie Geräte mit Nahbereichskommunikationstechnologien wie NFC, RFID, Bluetooth, Bluetooth LE, ZigBee, Infrarot (z. B. IrDA) oder Ähnlichem verbinden.

Die Benutzerschnittstellen können eine Anzeige (31) umfassen. Eine Anzeige (31) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (11, 12) kann/können verdrahtet oder drahtlos sein und kann/können so konfiguriert sein, dass sie Informationen von einem Benutzer in das Computersystem (1) empfängt/empfangen, z.B. zur Verarbeitung, Speicherung und/oder Anzeige. Geeignete Beispiele für Benutzereingabeschnittstellen sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen können ferner eine oder mehrere Schnittstellen für die Kommunikation mit Peripheriegeräten wie Druckern und dergleichen umfassen.

Ein oder mehrere Computerprogramme (40) können im Speicher (22) gespeichert sein und von der Verarbeitungseinheit (21) ausgeführt werden, die dadurch programmiert wird, die in dieser Beschreibung beschriebenen Funktionen zu erfüllen. Das Abrufen, Laden und Ausführen von Anweisungen des Computerprogramms (40) kann sequenziell erfolgen, so dass jeweils ein Befehl abgerufen, geladen und ausgeführt wird. Das Abrufen, Laden und/oder Ausführen kann aber auch parallel erfolgen.

In dem Speicher (22) kann auch das erfindungsgemäße Modell des maschinellen Lernens gespeichert sein.

Das erfindungsgemäße System kann als Laptop, Notebook, Netbook und/der Tablet-PC ausgeführt sein, es kann auch ein Bestandteil eines MRT-Scanners, eines CT-Scanners oder eines Ultraschalldiagnosegeräts sein.

**Patentansprüche**

1. Computer-implementiertes Verfahren umfassend die Schritte:

   - Empfangen von Trainingsdaten, wobei die Trainingsdaten eine Vielzahl von Datensätzen einer Vielzahl von Referenz-Untersuchungsobjekten umfassen, wobei die Datensätze Referenz-Repräsentationen eines Untersuchungsbereichs umfassen, wobei der Untersuchungsbereich die Leber oder ein Teil der Leber eines Menschen ist, wobei die Referenz-Repräsentationen radiologische Aufnahmen sind,

   wobei jeder Datensatz eine oder mehrere Referenz-Repräsentationen einer Zahl $n$ an Referenz-Repräsentationen umfasst, wobei $n$ eine ganze Zahl größer als 2 ist,
   wobei jede Referenz-Repräsentation $R_i$ den Untersuchungsbereich in einem Zustand $Z_i$ repräsentiert, wobei $i$ ein Index ist, der die Zahlen von 1 bis $n$ durchläuft,
   wobei die Zustände $Z_1, ..., Z_n$ eine Folge von Zuständen bilden,

   - Trainieren des Modells des maschinellen Lernens,

     ∘ wobei das Modell trainiert wird, ausgehend von der Referenz-Repräsentation $R_1$ nacheinander vorhergesagte Repräsentationen $R_2^*$ bis $R_n^*$ zu erzeugen,
     ∘ wobei die Erzeugung der vorhergesagten Repräsentation $R_2^*$ zumindest anteilig auf Basis der Referenz-Repräsentation $R_1$ erfolgt und die Erzeugung jeder nachfolgenden vorhergesagten Repräsentation $R_k^*$ zumindest anteilig auf Basis der vorhergesagten Repräsentation $R_{k-1}^*$ erfolgt, wobei $k$ ein Index ist, der die Zahlen von 3 bis $n$ durchläuft,
     ∘ wobei die vorhergesagte Repräsentation $R_2^*$ durch Eingabe der Referenz-Repräsentation $R_1$ in das Modell während des Trainings erzeugt wird, und jede nachfolgende vorhergesagte Repräsentation $R_k^*$ durch Eingabe der vorhergesagten Repräsentation $R_{k-1}^*$ in das Modell während des Trainings erzeugt wird,
     ∘ wobei die vorhergesagte Repräsentation $R_k^*$ den Untersuchungsbereich im Zustand $Z_k$ repräsentiert und die vorhergesagte Repräsentation $R_{k-1}^*$ den Untersuchungsbereich im Zustand $Z_{k-1}$ repräsentiert, und der Zustand $Z_{k-1}$ dem Zustand $Z_k$ unmittelbar vorangeht,

wobei das Trainieren des Modells des maschinellen Lernens ferner die Schritte umfasst:

- für jede vorhergesagte Repräsentation $R_j^*$: Berechnen eines Fehlerwerts für jedes Paar aus einer Referenz-Repräsentation $R_j$ und der vorhergesagten Repräsentation $R_j^*$ mit Hilfe einer Fehlerfunktion, wobei $j$ ein Index ist, der die Zahlen von 2 bis $n$ durchläuft,
- Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
- Modifizieren von Parametern des Modells des maschinellen Lernens, so dass der Gesamtfehlerwert auf ein definiertes Minimum reduziert wird,

- Speichern des trainierten Modells des maschinellen Lernens und/oder Nutzen des Modells des maschinellen Lernens zur Vorhersage,

wobei die Folge von Zuständen einen oder mehrere der folgenden Zustände umfasst

- die Leber oder einen Teil davon vor der Applikation eines hepatobiliären Kontrastmittels,
- die Leber oder einen Teil davon während der arteriellen Phase nach der Applikation des hepatobiliären Kontrastmittels,
- die Leber oder einen Teil davon während der portalvenösen Phase nach der Applikation des hepatobiliären Kontrastmittels,
- die Leber oder einen Teil davon während der Übergangsphase nach der Applikation des hepatobiliären Kontrastmittels,
- die Leber oder einen Teil davon während der hepatobiliären Phase nach der Applikation des hepatobiliären Kontrastmittels.

2. Verfahren gemäß Anspruch 1, ferner umfassend die Schritte:

• Empfangen einer Repräsentation $R_p$ des Untersuchungsbereichs, wobei die Repräsentation $R_p$ den Untersuchungsbereich in einem Zustand $Z_p$ der Folge von Zuständen $Z_1$ bis $Z_n$ repräsentiert, wobei $p$ eine ganze Zahl ist, die kleiner als $n$ ist,
• Zuführen der Repräsentation $R_p$ dem trainierten Modell des maschinellen Lernens,
• Empfangen, von dem Modell des maschinellen Lernens, einer oder mehrerer vorhergesagter Repräsentationen $R_{p+q}^*$ des Untersuchungsbereichs, wobei jede der einen oder der mehreren vorhergesagten Repräsentationen $R_{p+q}^*$ den Untersuchungsbereich in einem Zustand $Z_{p+q}^*$ repräsentiert, wobei q ein Index ist, der die Zahlen von 1 bis $m$ durchläuft, wobei $m$ eine ganze Zahl ist, die kleiner als $n$-1 ist oder gleich $n$-1 ist oder größer als $n$-1 ist.
• Ausgeben und/oder Speichern und/oder Übermitteln der einen oder der mehreren vorhergesagten Repräsentationen $R_{p+q}^*$.

3. Verfahren gemäß Anspruch 2, wobei jede von dem Modell des maschinellen Lernens erzeugte vorhergesagte Repräsentation $R_{p+q}^*$ wieder dem trainierten Modell des maschinellen Lernens zugeführt wird, um eine vorhergesagte Repräsentation $R_{p+q+1}^*$ zu erzeugen.

4. Verfahren gemäß Anspruch 3, wobei $m$ im Bereich von $n$ bis $n+2$ liegt.

5. Verfahren gemäß einem der Ansprüche 1 bis 4, wobei es sich bei den Referenz-Repräsentationen und den vorhergesagten Repräsentationen und der Repräsentation $R_p$ und den vorhergesagten Repräsentationen $R_{p+q}^*$ und $R_{p+q+1}^*$ um CT-Aufnahmen, MRT-Aufnahmen oder Ultraschallaufnahmen handelt.

6. Verfahren gemäß Anspruch 1, wobei die Gesamtfehlerfunktion folgende Formel hat:

$$LV = \sum_{j=2}^{n} w_j \cdot LF_j$$

wobei LV der Gesamtfehlerwert ist, wobei $LF_j$ die Fehlerfunktionen für die Berechnung der Fehlerwerte für die Abweichungen zwischen der Referenz-Repräsentation $R_j$ und der vorhergesagten Repräsentation $R_j^*$ ist, und $w_j$ die Gewichtsfaktoren sind, wobei $j$ ein Index ist, der die Zahlen von 2 bis $n$ durchläuft.

7. Verfahren gemäß einem der Ansprüche 1 bis 6, wobei jede vorhergesagte Repräsentation $R_{p+q}^*$ nach der folgenden

Formel berechnet wird:

$$\mathbf{M}^q\,(R_1) = R_{1+q}{}^*$$

wobei **M** das Modell des maschinellen Lernens repräsentiert, wobei $\mathbf{M}^q$ die q-fache Anwendung des Modells des maschinellen Lernens bedeutet, und $q$ eine ganze Zahl ist, die die Werte 1 bis $n$-1 annehmen kann.

8. Verfahren gemäß einem der Ansprüche 1 bis 7, wobei die Folge von Zuständen einen oder mehrere der folgenden Zustände umfasst:

   - einen ersten Zustand des Untersuchungsbereichs zu einem ersten Zeitpunkt vor der Applikation eines Kontrastmittels,
   - einen zweiten Zustand des Untersuchungsbereichs zu einem zweiten Zeitpunkt, nach der Applikation des Kontrastmittels,
   - einen dritten Zustand des Untersuchungsbereichs zu einem dritten Zeitpunkt, nach der Applikation des Kontrastmittels, sein,
   - einen vierten Zustand des Untersuchungsbereichs zu einem vierten Zeitpunkt, nach der Applikation des Kontrastmittels,
   - einen fünften Zustand des Untersuchungsbereichs zu einem fünften Zeitpunkt, nach der Applikation des Kontrastmittels,

   wobei der erste Zeitpunkt, der zweite Zeitpunkt, der dritte Zeitpunkt, der vierte Zeitpunkt und der fünfte Zeitpunkt eine zeitliche Folge bilden.

9. Computersystem umfassend

   • eine Eingabeeinheit,
   • eine Steuer- und Recheneinheit und
   • eine Ausgabeeinheit,
   wobei die Steuer- und Recheneinheit konfiguriert ist,
   • eine Repräsentation $R_p$ eines Untersuchungsbereichs zu empfangen, wobei die Repräsentation $R_p$ den Untersuchungsbereich in einem Zustand $Z_p$ einer Folge von Zuständen $Z_1$ bis $Z_n$ repräsentiert, wobei $p$ eine ganze Zahl ist, die kleiner als $n$ ist, wobei $n$ eine ganze Zahl ist, die größer als zwei ist, wobei der Untersuchungsbereich die Leber oder ein Teil der Leber eines Menschen ist, wobei die Repräsentation $R_p$ eine radiologische Aufnahme ist,
   • die Repräsentation $R_p$ einem trainierten Modell des maschinellen Lernens zuzuführen,

      ○ wobei das Modell des maschinellen Lernens auf Basis von Trainingsdaten trainiert worden ist, ausgehend von einer ersten Referenz-Repräsentation $R_1$ eine Zahl $n$-1 von vorhergesagten Repräsentationen $R_2{}^*$ bis $R_n{}^*$ zu erzeugen,
      ○ wobei die erste Referenz-Repräsentation $R_1$ den Untersuchungsbereich in einem ersten Zustand $Z_1$ repräsentiert und jede vorhergesagte Repräsentation $R_j{}^*$ den Untersuchungsbereich im Zustand $Z_j$ reprä-sentiert, wobei $j$ ein Index ist, der die Zahlen von 2 bis $n$ durchläuft,
      ○ wobei die Erzeugung der vorhergesagten Repräsentation $R_2{}^*$ zumindest anteilig auf Basis der Referenz-Repräsentation $R_1$ erfolgt und die Erzeugung jeder weiteren vorhergesagten Repräsentation $R_k{}^*$ zumindest anteilig auf Basis der vorhergesagten Repräsentation $R_{k-1}{}^*$ erfolgt, wobei $k$ ein Index ist, der die Zahlen von 3 bis $n$ durchläuft,
      ○ wobei die vorhergesagte Repräsentation $R_2{}^*$ durch Eingabe der Referenz-Repräsentation $R_1$ in das Modell während des Trainings erzeugt wird, und jede nachfolgende vorhergesagte Repräsentation $R_k{}^*$ durch Eingabe der vorhergesagten Repräsentation $R_{k-1}{}^*$ in das Modell während des Trainings erzeugt wird,
      ○ wobei das Trainieren des Modells des maschinellen Lernens ferner die Schritte umfasst:

         - für jede vorhergesagte Repräsentation $R_j{}^*$: Berechnen eines Fehlerwerts für jedes Paar aus einer Referenz-Repräsentation $R_j$ und der vorhergesagten Repräsentation $R_j{}^*$ mit Hilfe einer Fehlerfunktion, wobei $j$ ein Index ist, der die Zahlen von 2 bis $n$ durchläuft,
         - Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,

- Modifizieren von Parametern des Modells des maschinellen Lernens, so dass der Gesamtfehlerwert auf ein definiertes Minimum reduziert wird,

• von dem Modell des maschinellen Lernens eine oder mehrere vorhergesagte Repräsentationen $R_{p+q}^*$ des Untersuchungsbereichs zu empfangen, wobei jede der einen oder der mehreren vorhergesagten Repräsentationen $R_{p+q}^*$ den Untersuchungsbereich in einem Zustand $Z_{p+q}^*$ repräsentiert, wobei $q$ ein Index ist, der die Zahlen von 1 bis $m$ durchläuft, wobei $m$ eine ganze Zahl ist, die kleiner als $n$-1 ist oder gleich $n$-1 ist oder größer als $n$-1 ist,

die eine oder die mehreren vorhergesagten Repräsentationen $R_{p+q}^*$ auszugeben und/oder zu speichern und/oder an ein separates Computersystem zu übermitteln,

wobei die Folge von Zuständen einen oder mehrere der folgenden Zustände umfasst:

- die Leber oder einen Teil davon vor der Applikation eines hepatobiliären Kontrastmittels,
- die Leber oder einen Teil davon während der arteriellen Phase nach der Applikation des hepatobiliären Kontrastmittels,
- die Leber oder einen Teil davon während der portalvenösen Phase nach der Applikation des hepatobiliären Kontrastmittels,
- die Leber oder einen Teil davon während der Übergangsphase nach der Applikation des hepatobiliären Kontrastmittels,
- die Leber oder einen Teil davon während der hepatobiliären Phase nach der Applikation des hepatobiliären Kontrastmittels.

10. Computerprogrammprodukt umfassend ein Computerprogramm, das in einen Arbeitsspeicher eines Computersystems geladen werden kann und dort das Computersystem dazu veranlasst, folgende Schritte ausführen:

• Empfangen einer Repräsentation $R_p$ eines Untersuchungsbereichs, wobei die Repräsentation $R_p$ den Untersuchungsbereich in einem Zustand $Z_p$ einer Folge von Zuständen $Z_1$ bis $Z_n$ repräsentiert, wobei $p$ eine ganze Zahl ist, die kleiner als $n$ ist, wobei $n$ eine ganze Zahl ist, die größer als zwei ist, wobei der Untersuchungsbereich die Leber oder ein Teil der Leber eines Menschen ist, wobei die Repräsentation $R_p$ eine radiologische Aufnahme ist,
• Zuführen der Repräsentation $R_p$ einem trainierten Modell des maschinellen Lernens,

○ wobei das Modell des maschinellen Lernens auf Basis von Trainingsdaten trainiert worden ist, ausgehend von einer ersten Referenz-Repräsentation $R_1$ eine Zahl $n$-1 von vorhergesagten Repräsentationen $R_2^*$ bis $R_n^*$ zu erzeugen,
○ wobei die erste Referenz-Repräsentation $R_1$ den Untersuchungsbereich in einem ersten Zustand $Z_1$ repräsentiert und jede vorhergesagte Repräsentation $R_j^*$ den Untersuchungsbereich im Zustand $Z_j$ repräsentiert, wobei $j$ ein Index ist, der die Zahlen von 2 bis $n$ durchläuft,
○ wobei die Erzeugung der vorhergesagten Repräsentation $R_2^*$ zumindest anteilig auf Basis der Referenz-Repräsentation $R_1$ erfolgt und die Erzeugung jeder weiteren vorhergesagten Repräsentation $R_k^*$ zumindest anteilig auf Basis der vorhergesagten Repräsentation $R_{k-1}^*$ erfolgt, wobei $k$ ein Index ist, der die Zahlen von 3 bis $n$ durchläuft,
○ wobei die vorhergesagte Repräsentation $R_2^*$ durch Eingabe der Referenz-Repräsentation $R_1$ in das Modell während des Trainings erzeugt wird, und jede nachfolgende vorhergesagte Repräsentation $R_k^*$ durch Eingabe der vorhergesagten Repräsentation $R_{k-1}^*$ in das Modell während des Trainings erzeugt wird,
○ wobei das Trainieren des Modells des maschinellen Lernens ferner die Schritte umfasst:

- für jede vorhergesagte Repräsentation $R_j^*$: Berechnen eines Fehlerwerts für jedes Paar aus einer Referenz-Repräsentation $R_j$ und der vorhergesagten Repräsentation $R_j^*$ mit Hilfe einer Fehlerfunktion, wobei $j$ ein Index ist, der die Zahlen von 2 bis $n$ durchläuft,
- Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
- Modifizieren von Parametern des Modells des maschinellen Lernens, so dass der Gesamtfehlerwert auf ein definiertes Minimum reduziert wird,

• Empfangen, von dem Modell des maschinellen Lernens, einer oder mehrerer vorhergesagter Repräsentationen $R_{p+q}^*$ des Untersuchungsbereichs, wobei jede der einen oder der mehreren vorhergesagten Repräsentationen $R_{p+q}^*$ den Untersuchungsbereich in einem Zustand $Z_{p+q}^*$ repräsentiert, wobei $q$ ein Index ist, der die Zahlen von 1 bis $m$ durchläuft, wobei $m$ eine ganze Zahl ist, die kleiner als $n$-1 ist oder gleich $n$-1 ist oder größer als $n$-1 ist.

• Ausgeben und/oder Speichern und/oder Übermitteln der einen oder der mehreren vorhergesagten Repräsentationen $R_{p+q}*$,

wobei die Folge von Zuständen einen oder mehrere der folgenden Zustände umfasst:

• die Leber oder einen Teil davon vor der Applikation eines hepatobiliären Kontrastmittels,
• die Leber oder einen Teil davon während der arteriellen Phase nach der Applikation des hepatobiliären Kontrastmittels,
• die Leber oder einen Teil davon während der portalvenösen Phase nach der Applikation des hepatobiliären Kontrastmittels,
• die Leber oder einen Teil davon während der Übergangsphase nach der Applikation des hepatobiliären Kontrastmittels,
• die Leber oder einen Teil davon während der hepatobiliären Phase nach der Applikation des hepatobiliären Kontrastmittels.

11. Verwendung eines Kontrastmittels in einem radiologischen Untersuchungsverfahren, wobei das radiologische Untersuchungsverfahren die folgenden Schritte umfasst:

• Empfangen einer Repräsentation $R_p$ eines Untersuchungsbereichs, wobei die Repräsentation $R_p$ den Untersuchungsbereich vor oder nach Applikation des Kontrastmittels in einem Zustand $Z_p$ einer Folge von Zuständen $Z_1$ bis $Z_n$ repräsentiert, wobei $p$ eine ganze Zahl ist, die kleiner als $n$ ist, wobei $n$ eine ganze Zahl ist, die größer als zwei ist, wobei der Untersuchungsbereich die Leber oder ein Teil der Leber eines Menschen ist, wobei die Repräsentation $R_p$ eine radiologische Aufnahme ist,
• Zuführen der Repräsentation $R_p$ einem trainierten Modell des maschinellen Lernens,

   o wobei das Modell des maschinellen Lernens auf Basis von Trainingsdaten trainiert worden ist, ausgehend von einer ersten Referenz-Repräsentation $R_1$ eine Zahl $n-1$ von vorhergesagten Repräsentationen $R_2*$ bis $R_n*$ zu erzeugen,
   ◦ wobei die erste Referenz-Repräsentation $R_1$ den Untersuchungsbereich in einem ersten Zustand $Z_1$ repräsentiert und jede vorhergesagte Repräsentation $R_j*$ den Untersuchungsbereich im Zustand $Z_j$ repräsentiert, wobei $j$ ein Index ist, der die Zahlen von 2 bis $n$ durchläuft,
   o wobei die Erzeugung der vorhergesagten Repräsentation $R_2*$ zumindest anteilig auf Basis der Referenz-Repräsentation $R_1$ erfolgt und die Erzeugung jeder weiteren vorhergesagten Repräsentation $R_k*$ zumindest anteilig auf Basis der vorhergesagten Repräsentation $R_{k-1}*$ erfolgt, wobei $k$ ein Index ist, der die Zahlen von 3 bis $n$ durchläuft,
   o wobei die vorhergesagte Repräsentation $R_2*$ durch Eingabe der Referenz-Repräsentation $R_1$ in das Modell während des Trainings erzeugt wird, und jede nachfolgende vorhergesagte Repräsentation $R_k*$ durch Eingabe der vorhergesagten Repräsentation $R_{k-1}*$ in das Modell während des Trainings erzeugt wird,
   ◦ wobei das Trainieren des Modells des maschinellen Lernens ferner die Schritte umfasst:

      - für jede vorhergesagte Repräsentation $R_j*$: Berechnen eines Fehlerwerts für jedes Paar aus einer Referenz-Repräsentation $R_j$ und der vorhergesagten Repräsentation $R_j*$ mit Hilfe einer Fehlerfunktion, wobei $j$ ein Index ist, der die Zahlen von 2 bis $n$ durchläuft,
      - Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
      - Modifizieren von Parametern des Modells des maschinellen Lernens, so dass der Gesamtfehlerwert auf ein definiertes Minimum reduziert wird,

• Empfangen, von dem Modell des maschinellen Lernens, einer oder mehrerer vorhergesagter Repräsentationen $R_{p+q}*$ des Untersuchungsbereichs, wobei jede der einen oder der mehreren vorhergesagten Repräsentationen $R_{p+q}*$ den Untersuchungsbereich in einem Zustand $Z_{p+q}*$ repräsentiert, wobei $q$ ein Index ist, der die Zahlen von 1 bis $m$ durchläuft, wobei $m$ eine ganze Zahl ist, die kleiner als $n-1$ ist oder gleich $n-1$ ist oder größer als $n-1$ ist.
• Ausgeben und/oder Speichern und/oder Übermitteln der einen oder der mehreren vorhergesagten Repräsentationen $R_{p+q}*$,

wobei die Folge von Zuständen einen oder mehrere der folgenden Zustände umfasst:

• die Leber oder einen Teil davon vor der Applikation eines hepatobiliären Kontrastmittels,

• die Leber oder einen Teil davon während der arteriellen Phase nach der Applikation des hepatobiliären Kontrastmittels,
• die Leber oder einen Teil davon während der portalvenösen Phase nach der Applikation des hepatobiliären Kontrastmittels,
• die Leber oder einen Teil davon während der Übergangsphase nach der Applikation des hepatobiliären Kontrastmittels,
• die Leber oder einen Teil davon während der hepatobiliären Phase nach der Applikation des hepatobiliären Kontrastmittels.

12. Kit umfassend ein Kontrastmittel und ein Computerprogrammprodukt, wobei das Computerprogrammprodukt ein Computerprogramm umfasst, das in einen Arbeitsspeicher eines Computersystems geladen werden kann und dort das Computersystem dazu veranlasst, folgende Schritte ansführen:

• Empfangen einer Repräsentation $R_p$ eines Untersuchungsbereichs, wobei die Repräsentation $R_p$ den Untersuchungsbereich vor oder nach Applikation des Kontrastmittels in einem Zustand $Z_p$ einer Folge von Zuständen $Z_1$ bis $Z_n$ repräsentiert, wobei $p$ eine ganze Zahl ist, die kleiner als $n$ ist, wobei $n$ eine ganze Zahl ist, die größer als zwei ist, wobei der Untersuchungsbereich die Leber oder ein Teil der Leber eines Menschen ist, wobei die Repräsentation $R_p$ eine radiologische Aufnahme ist,
• Zuführen der Repräsentation $R_p$ einem trainierten Modell des maschinellen Lernens,

   o wobei das Modell des maschinellen Lernens auf Basis von Trainingsdaten trainiert worden ist, ausgehend von einer ersten Referenz-Repräsentation $R_1$ eine Zahl $n-1$ von vorhergesagten Repräsentationen $R_2^*$ bis $R_n^*$ zu erzeugen,
   ◦ wobei die erste Referenz-Repräsentation $R_1$ den Untersuchungsbereich in einem ersten Zustand $Z_1$ repräsentiert und jede vorhergesagte Repräsentation $R_j^*$ den Untersuchungsbereich im Zustand $Z_j$ repräsentiert, wobei $j$ ein Index ist, der die Zahlen von 2 bis $n$ durchläuft,
   o wobei die Erzeugung der vorhergesagten Repräsentation $R_2^*$ zumindest anteilig auf Basis der Referenz-Repräsentation $R_1$ erfolgt und die Erzeugung jeder weiteren vorhergesagten Repräsentation $R_k^*$ zumindest anteilig auf Basis der vorhergesagten Repräsentation $R_{k-1}^*$ erfolgt, wobei $k$ ein Index ist, der die Zahlen von 3 bis $n$ durchläuft,
   o wobei die vorhergesagte Repräsentation $R_2^*$ durch Eingabe der Referenz-Repräsentation $R_1$ in das Modell während des Trainings erzeugt wird, und jede nachfolgende vorhergesagte Repräsentation $R_k^*$ durch Eingabe der vorhergesagten Repräsentation $R_{k-1}^*$ in das Modell während des Trainings erzeugt wird,
   ◦ wobei das Trainieren des Modells des maschinellen Lernens ferner die Schritte umfasst:

      - für jede vorhergesagte Repräsentation $R_j^*$: Berechnen eines Fehlerwerts für jedes Paar aus einer Referenz-Repräsentation $R_j$ und der vorhergesagten Repräsentation $R_j^*$ mit Hilfe einer Fehlerfunktion, wobei $j$ ein Index ist, der die Zahlen von 2 bis $n$ durchläuft,
      - Berechnen eines Gesamtfehlerwerts mit Hilfe einer Gesamtfehlerfunktion, wobei die Gesamtfehlerfunktion eine Funktion der Fehlerwerte ist, in der die Fehlerwerte mit Gewichtsfaktoren gewichtet sind,
      - Modifizieren von Parametern des Modells des maschinellen Lernens, so dass der Gesamtfehlerwert auf ein definiertes Minimum reduziert wird,

• Empfangen, von dem Modell des maschinellen Lernens, einer oder mehrerer vorhergesagter Repräsentationen $R_{p+q}^*$ des Untersuchungsbereichs, wobei jede der einen oder der mehreren vorhergesagten Repräsentationen $R_{p+q}^*$ den Untersuchungsbereich in einem Zustand $Z_{p+q}^*$ repräsentiert, wobei $q$ ein Index ist, der die Zahlen von 1 bis $m$ durchläuft, wobei $m$ eine ganze Zahl ist, die kleiner als $n-1$ ist oder gleich $n-1$ ist oder größer als $n-1$ ist.
• Ausgeben und/oder Speichern und/oder Übermitteln der einen oder der mehreren vorhergesagten Repräsentationen $R_{p+q}^*$,

wobei die Folge von Zuständen einen oder mehrere der folgenden Zustände umfasst

• die Leber oder einen Teil davon vor der Applikation eines hepatobiliären Kontrastmittels,
• die Leber oder einen Teil davon während der arteriellen Phase nach der Applikation des hepatobiliären Kontrastmittels,
• die Leber oder einen Teil davon während der portalvenösen Phase nach der Applikation des hepatobiliären Kontrastmittels,
• die Leber oder einen Teil davon während der Übergangsphase nach der Applikation des hepatobiliären Kontrastmittels,
• die Leber oder einen Teil davon während der hepatobiliären Phase nach der Applikation des hepatobiliären Kontrastmittels.

**Claims**

1. Computer-implemented method comprising the steps of:

   - receiving training data, where the training data comprise a multiplicity of data sets of a multiplicity of reference examination objects, where the data sets comprise reference representations of an examination region, where the examination region is the liver or part of the liver of a human, where the reference representations are radiological images,

     where each data set comprises one or more reference representations of a number $n$ of reference representations, where $n$ is an integer greater than 2, where each reference representation $R_i$ represents the examination region in one state $Z_i$, where $i$ is an index passing through the numbers from 1 to $n$, where the states $Z_1$, ..., $Z_n$ form a sequence of states,

   - training the machine-learning model,

     ○ where the model is trained to generate, starting from the reference representation $R_1$, predicted representations $R_2^*$ to $R_n^*$ one after the other,
     ○ where the predicted representation $R_2^*$ is generated at least partly on the basis of the reference representation $R_1$ and each subsequent predicted representation $R_k^*$ is generated at least partly on the basis of the predicted representation $R_{k-1}^*$, where $k$ is an index passing through the numbers from 3 to $n$,
     ○ where the predicted representation $R_2^*$ is generated by inputting the reference representation $R_1$ into the model during training, and each subsequent predicted representation $R_k^*$ is generated by inputting the predicted representation $R_{k-1}^*$ into the model during training,
     ○ where the predicted representation $R_k^*$ represents the examination region in the state $Z_k$ and the predicted representation $R_{k-1}^*$ represents the examination region in the state $Z_{k-1}$, and the state $2_{k-1}$ directly precedes the state $Z_k$,

     where training the machine-learning model further comprises the steps of:

     - for each predicted representation $R_j^*$: calculating a loss value for each pair composed of a reference representation $R_j$ and the predicted representation $R_j^*$ with the aid of a loss function, where $j$ is an index passing through the numbers from 2 to $n$,
     - calculating a total loss value with the aid of a total loss function, where the total loss function is a function of the loss values in which the loss values are weighted with weight factors,
     - modifying parameters of the machine-learning model, such that the total loss value is reduced to a defined minimum,
     - storing the trained machine-learning model and/or utilizing the machine-learning model for prediction,

     where the sequence of states comprises one or more of the following states:

     - the liver or part of the liver before the administration of a hepatobiliary contrast agent,
     - the liver or part of the liver during the arterial phase after the administration of the hepatobiliary contrast agent,
     - the liver or part of the liver during the portal venous phase after the administration of the hepatobiliary contrast agent,
     - the liver or part of the liver during the transitional phase after the administration of the hepatobiliary contrast agent,
     - the liver or part of the liver during the hepatobiliary phase after the administration of the hepatobiliary contrast agent.

2. Method according to Claim 1, further comprising the steps of:

• receiving a representation $R_p$ of the examination region, where the representation $R_p$ represents the examination region in one state $Z_p$ of the sequence of states $Z_1$ to $Z_n$, where $p$ is an integer less than n,
• feeding the representation $R_p$ to the trained machine-learning model,
• receiving from the machine-learning model one or more predicted representations $R_{p+q}^*$ of the examination region, where each of the one or more predicted representations $R_{p+q}^*$ represents the examination region in one state $Z_{p+q}^*$, where $q$ is an index passing through the numbers from 1 to $m$, where $m$ is an integer less than $n$-1 or equal to $n$-1 or greater than $n$-1,
• outputting and/or storing and/or transmitting the one or more predicted representations $R_{p+q}^*$.

3. Method according to Claim 2, wherein each predicted representation $R_{p+q}^*$ generated by the machine-learning model is fed back to the trained machine-learning model in order to generate a predicted representation $R_{p+q+1}^*$.

4. Method according to Claims 3, wherein $m$ is in the range from $n$ to $n$+2.

5. Method according to any of Claims 1 to 4, wherein the reference representations and the predicted representations and the representation $R_p$ and the predicted representations $R_{p+q}^*$ and $R_{p+q+1}^*$ are CT images, MRI images or ultrasound images.

6. Method according to Claim 1, wherein the total loss function has the following formula:

$$LV = \sum_{j=2}^{n} w_j \cdot LF_j$$

where LV is the total loss value, where $LF_j$ are the loss functions for calculating the loss values for the differences between the reference representation $R_j$ and the predicted representation $R_j^*$, and $w_j$ are weight factors, where $j$ is an index passing through the numbers from 2 to $n$.

7. Method according to any of Claims 1 to 6, wherein each predicted representation $R_{p+q}^*$ is calculated according to the following formula:

$$\mathbf{M}^q (R_1) = R_{1+q}^*$$

where M represents the machine-learning model, where $\mathbf{M}^q$ means the $q$-times application of the machine-learning model, and $q$ is an integer which can assume the values of 1 to $n$-1.

8. Method according to any of Claims 1 to 7, wherein the sequence of states comprises one or more of the following states:

- a first state of the examination region at a first time point before the administration of a contrast agent,
- a second state of the examination region at a second time point, after the administration of the contrast agent,
- a third state of the examination region at a third time point, after the administration of the contrast agent,
- a fourth state of the examination region at a fourth time point, after the administration of the contrast agent,
- a fifth state of the examination region at a fifth time point, after the administration of the contrast agent,

where the first time point, the second time point, the third time point, the fourth time point and the fifth time point form a chronological sequence.

9. Computer system comprising

• an input unit,
• a control and calculation unit and
• an output unit,
wherein the control and calculation unit is configured
• to receive a representation $R_p$ of an examination region, where the representation $R_p$ represents the examination region in one state $Z_p$ of a sequence of states $Z_1$ to $Z_n$, where $p$ is an integer less than n, where n is an integer greater than two, where the examination region is the liver or part of the liver of a human, where the representation $R_p$ is a radiological image,

• to feed the representation $R_p$ to a trained machine-learning model,

  ◦ where the machine-learning model has been trained on the basis of training data to generate, starting from a first reference representation $R_1$, a number $n$-1 of predicted representations $R_2^*$ to $R_n^*$,
  ◦ where the first reference representation $R_1$ represents the examination region in a first state $Z_1$ and each predicted representation $R_j^*$ represents the examination region in the state $Z_j$, where $j$ is an index passing through the numbers from 2 to $n$,
  ◦ where the predicted representation $R_2^*$ is generated at least partly on the basis of the reference representation $R_1$ and each further predicted representation $R_k^*$ is generated at least partly on the basis of the predicted representation $R_{k-1}^*$, where $k$ is an index passing through the numbers from 3 to $n$,
  ◦ where the predicted representation $R_2^*$ is generated by inputting the reference representation $R_1$ into the model during training, and each subsequent predicted representation $R_k^*$ is generated by inputting the predicted representation $R_{k-1}^*$ into the model during training,
  ◦ where training the machine-learning model further comprises the steps of:

    - for each predicted representation $R_j^*$: calculating a loss value for each pair composed of a reference representation $R_j$ and the predicted representation $R_j^*$ with the aid of a loss function, where $j$ is an index passing through the numbers from 2 to $n$,
    - calculating a total loss value with the aid of a total loss function, where the total loss function is a function of the loss values in which the loss values are weighted with weight factors,
    - modifying parameters of the machine-learning model, such that the total loss value is reduced to a defined minimum,

• to receive from the machine-learning model one or more predicted representations $R_{p+q}^*$ of the examination region, where each of the one or more predicted representations $R_{p+q}^*$ represents the examination region in one state $Z_{p+q}^*$, where $q$ is an index passing through the numbers from 1 to $m$, where $m$ is an integer less than $n$-1 or equal to $n$-1 or greater than $n$-1,
to output the one or more predicted representations $R_{p+q}^*$ and/or to store them and/or to transmit them to a separate computer system,
where the sequence of states comprises one or more of the following states:

  - the liver or part of the liver before the administration of a hepatobiliary contrast agent,
  - the liver or part of the liver during the arterial phase after the administration of the hepatobiliary contrast agent,
  - the liver or part of the liver during the portal venous phase after the administration of the hepatobiliary contrast agent,
  - the liver or part of the liver during the transitional phase after the administration of the hepatobiliary contrast agent,
  - the liver or part of the liver during the hepatobiliary phase after the administration of the hepatobiliary contrast agent.

10. Computer program product comprising a computer program which can be loaded into a working memory of a computer system, where it causes the computer system to execute the following steps:

• receiving a representation $R_p$ of an examination region, where the representation $R_p$ represents the examination region in one state $Z_p$ of a sequence of states $Z_1$ to $Z_n$, where $p$ is an integer less than $n$, where $n$ is an integer greater than two, where the examination region is the liver or part of the liver of a human, where the representation $R_p$ is a radiological image,
• feeding the representation $R_p$ to a trained machine-learning model,

  ◦ where the machine-learning model has been trained on the basis of training data to generate, starting from a first reference representation $R_1$, a number $n$-1 of predicted representations $R_2^*$ to $R_n^*$,
  ◦ where the first reference representation $R_1$ represents the examination region in a first state $Z_1$ and each predicted representation $R_j^*$ represents the examination region in the state $Z_j$, where $j$ is an index passing through the numbers from 2 to $n$,
  ◦ where the predicted representation $R_2^*$ is generated at least partly on the basis of the reference representation $R_1$ and each further predicted representation $R_k^*$ is generated at least partly on the basis of the predicted representation $R_{k-1}^*$, where $k$ is an index passing through the numbers from 3 to $n$,

◦ where the predicted representation $R_2^*$ is generated by inputting the reference representation $R_1$ into the model during training, and each subsequent predicted representation $R_k^*$ is generated by inputting the predicted representation $R_{k-1}^*$ into the model during training,

o where training the machine-learning model further comprises the steps of:

- for each predicted representation $R_j^*$: calculating a loss value for each pair composed of a reference representation $R_j$ and the predicted representation $R_j^*$ with the aid of a loss function, where $j$ is an index passing through the numbers from 2 to $n$,
- calculating a total loss value with the aid of a total loss function, where the total loss function is a function of the loss values in which the loss values are weighted with weight factors,
- modifying parameters of the machine-learning model, such that the total loss value is reduced to a defined minimum,

• receiving from the machine-learning model one or more predicted representations $R_{p+q}^*$ of the examination region, where each of the one or more predicted representations $R_{p+q}^*$ represents the examination region in one state $Z_{p+q}^*$, where $q$ is an index passing through the numbers from 1 to $m$, where $m$ is an integer less than $n$-1 or equal to $n$-1 or greater than $n$-1,
• outputting and/or storing and/or transmitting the one or more predicted representations $R_{p+q}^*$,

where the sequence of states comprises one or more of the following states:

• the liver or part of the liver before the administration of a hepatobiliary contrast agent,
• the liver or part of the liver during the arterial phase after the administration of the hepatobiliary contrast agent,
• the liver or part of the liver during the portal venous phase after the administration of the hepatobiliary contrast agent,
• the liver or part of the liver during the transitional phase after the administration of the hepatobiliary contrast agent,
• the liver or part of the liver during the hepatobiliary phase after the administration of the hepatobiliary contrast agent.

11. Use of a contrast agent in a radiological examination method, wherein the radiological examination method comprises the following steps:

• receiving a representation $R_p$ of an examination region, where the representation $R_p$ represents the examination region before or after administration of the contrast agent in one state $Z_p$ of a sequence of states $Z_1$ to $Z_n$, where $p$ is an integer less than $n$, where $n$ is an integer greater than two, where the examination region is the liver or part of the liver of a human, where the representation $R_p$ is a radiological image,
• feeding the representation $R_p$ to a trained machine-learning model,

◦ where the machine-learning model has been trained on the basis of training data to generate, starting from a first reference representation $R_1$, a number $n$-1 of predicted representations $R_2^*$ to $R_n^*$,
◦ where the first reference representation $R_1$ represents the examination region in a first state $Z_1$ and each predicted representation $R_j^*$ represents the examination region in the state $Z_j$, where $j$ is an index passing through the numbers from 2 to $n$,
◦ where the predicted representation $R_2^*$ is generated at least partly on the basis of the reference representation $R_1$ and each further predicted representation $R_k^*$ is generated at least partly on the basis of the predicted representation $R_{k-1}^*$, where $k$ is an index passing through the numbers from 3 to $n$,
◦ where the predicted representation $R_2^*$ is generated by inputting the reference representation $R_1$ into the model during training, and each subsequent predicted representation $R_k^*$ is generated by inputting the predicted representation $R_{k-1}^*$ into the model during training,

o where training the machine-learning model further comprises the steps of:

- for each predicted representation $R_j^*$: calculating a loss value for each pair composed of a reference representation $R_j$ and the predicted representation $R_j^*$ with the aid of a loss function, where $j$ is an index passing through the numbers from 2 to $n$,
- calculating a total loss value with the aid of a total loss function, where the total loss function is a function of the loss values in which the loss values are weighted with weight factors,
- modifying parameters of the machine-learning model, such that the total loss value is reduced to a defined minimum,

• receiving from the machine-learning model one or more predicted representations $R_{p+q}^*$ of the examination region, where each of the one or more predicted representations $R_{p+q}^*$ represents the examination region in one state $Z_{p+q}^*$, where $q$ is an index passing through the numbers from 1 to $m$, where $m$ is an integer less than $n$-1 or equal to $n$-1 or greater than $n$-1,

• outputting and/or storing and/or transmitting the one or more predicted representations $R_{p+q}^*$,

where the sequence of states comprises one or more of the following states:

• the liver or part of the liver before the administration of a hepatobiliary contrast agent,
• the liver or part of the liver during the arterial phase after the administration of the hepatobiliary contrast agent,
• the liver or part of the liver during the portal venous phase after the administration of the hepatobiliary contrast agent,
• the liver or part of the liver during the transitional phase after the administration of the hepatobiliary contrast agent,
• the liver or part of the liver during the hepatobiliary phase after the administration of the hepatobiliary contrast agent.

12. Kit comprising a contrast agent and a computer program product, wherein the computer program product comprises a computer program that can be loaded into a working memory of a computer system, where it causes the computer system to execute the following steps:

• receiving a representation $R_p$ of an examination region, where the representation $R_p$ represents the examination region before or after administration of the contrast agent in one state $Z_p$ of a sequence of states $Z_1$ to $Z_n$, where $p$ is an integer less than $n$, where $n$ is an integer greater than two, where the examination region is the liver or part of the liver of a human, where the representation $R_p$ is a radiological image,
• feeding the representation $R_p$ to a trained machine-learning model,

  ◦ where the machine-learning model has been trained on the basis of training data to generate, starting from a first reference representation $R_1$, a number $n$-1 of predicted representations $R_2^*$ to $R_n^*$,
  ◦ where the first reference representation $R_1$ represents the examination region in a first state $Z_1$ and each predicted representation $R_j^*$ represents the examination region in the state $Z_j$, where $j$ is an index passing through the numbers from 2 to $n$,
  ◦ where the predicted representation $R_2^*$ is generated at least partly on the basis of the reference representation $R_1$ and each further predicted representation $R_k^*$ is generated at least partly on the basis of the predicted representation $R_{k-1}^*$, where $k$ is an index passing through the numbers from 3 to $n$,
  ◦ where the predicted representation $R_2^*$ is generated by inputting the reference representation $R_1$ into the model during training, and each subsequent predicted representation $R_k^*$ is generated by inputting the predicted representation $R_{k-1}^*$ into the model during training,
  ◦ where training the machine-learning model further comprises the steps of:

    - for each predicted representation $R_j^*$: calculating a loss value for each pair composed of a reference representation $R_j$ and the predicted representation $R_j^*$ with the aid of a loss function, where $j$ is an index passing through the numbers from 2 to $n$,
    - calculating a total loss value with the aid of a total loss function, where the total loss function is a function of the loss values in which the loss values are weighted with weight factors,
    - modifying parameters of the machine-learning model, such that the total loss value is reduced to a defined minimum,

• receiving from the machine-learning model one or more predicted representations $R_{p+q}^*$ of the examination region, where each of the one or more predicted representations $R_{p+q}^*$ represents the examination region in one state $Z_{p+q}^*$, where $q$ is an index passing through the numbers from 1 to $m$, where $m$ is an integer less than $n$-1 or equal to $n$-1 or greater than $n$-1,
• outputting and/or storing and/or transmitting the one or more predicted representations $R_{p+q}^*$,

where the sequence of states comprises one or more of the following states:

• the liver or part of the liver before the administration of a hepatobiliary contrast agent,
• the liver or part of the liver during the arterial phase after the administration of the hepatobiliary contrast agent,
• the liver or part of the liver during the portal venous phase after the administration of the hepatobiliary contrast agent,
• the liver or part of the liver during the transitional phase after the administration of the hepatobiliary contrast agent,
• the liver or part of the liver during the hepatobiliary phase after the administration of the hepatobiliary contrast agent.

**Revendications**

1.  Procédé mis en œuvre par ordinateur, comprenant les étapes consistant à :

    - recevoir des données d'apprentissage, les données d'apprentissage comprenant une pluralité d'ensembles de données d'une pluralité d'objets d'examen de référence, les ensembles de données comprenant des représentations de référence d'une région d'examen, la région d'examen étant le foie ou une partie du foie d'un être humain, les représentations de référence étant des images radiologiques,

        chaque ensemble de données comprenant une ou plusieurs représentations de référence d'un nombre n de représentations de référence, n étant un entier supérieur à 2,
        chaque représentation de référence $R_i$ représente la région d'examen dans un état $R_i$, où i est un indice allant de 1 à n,
        les états $Z_1$, ..., $Z_n$ formant une séquence d'états,

    - entraîner le modèle d'apprentissage automatique,

        o le modèle étant entraîné pour générer successivement, à partir de la représentation de référence $R_1$, des représentations prédites $R_2^*$ à $R_n^*$,
        o la génération de la représentation prédite $R_2^*$ s'effectuant au moins proportionnellement sur la base de la représentation de référence $R_1$ et la génération de chaque représentation prédite suivante $R_k^*$ s'effectuant au moins proportionnellement sur la base de la représentation prédite $R_{k-1}^*$, où k est un indice allant de 3 à n,
        o la représentation prédite $R_2^*$ étant générée en fournissant en entrée la représentation de référence $R_1$ au modèle pendant l'entraînement, et chaque représentation prédite suivante $R_k^*$ étant générée en fournissant en entrée la représentation prédite $R_{k-1}^*$ au modèle pendant l'entraînement,
        o la représentation prédite $R_k^*$ représentant la région d'examen dans l'état $Z_k$ et la représentation prédite $R_{k-1}^*$ représentant la région d'examen dans l'état $Z_{k-1}$, et l'état $Z_{k-1}$ précède immédiatement l'état $Z_k$,

    l'entraînement du modèle d'apprentissage automatique comprenant en outre les étapes consistant à :

        - pour chaque représentation prédite $R_j^*$ : calculer une valeur d'erreur pour une paire constituée d'une représentation de référence $R_j$ et de la représentation prédite $R_j^*$ à l'aide d'une fonction d'erreur, où j est un indice allant de 2 à n,
        - calculer une valeur d'erreur totale à l'aide d'une fonction d'erreur totale, la fonction d'erreur totale étant une fonction des valeurs d'erreur dans lesquelles les valeurs d'erreur sont pondérées par des facteurs de pondération,
        - modifier les paramètres du modèle d'apprentissage automatique de telle sorte que la valeur d'erreur totale soit réduite à un minimum défini,
        - mémoriser le modèle d'apprentissage automatique entraîné et/ou utiliser le modèle d'apprentissage automatique pour la prédiction,

    la séquence d'états comprenant un ou plusieurs des états suivants :

        - le foie ou une partie de celui-ci avant l'application d'un agent de contraste hépatobiliaire,
        - le foie ou une partie de celui-ci pendant la phase artérielle après l'application de l'agent de contraste hépatobiliaire,
        - le foie ou une partie de celui-ci pendant la phase veineuse portale après l'application de l'agent de contraste hépatobiliaire,

- le foie ou une partie de celui-ci pendant la phase de transition après l'application de l'agent de contraste hépatobiliaire,
- le foie ou une partie de celui-ci pendant la phase hépatobiliaire après l'application de l'agent de contraste hépatobiliaire.

2. Procédé selon la revendication 1, comprenant les étapes consistant à :

• recevoir la représentation $R_p$ de la région d'examen, la représentation $R_p$ représentant la région d'examen dans un état $Z_p$ de la séquence d'états $Z_1$ à $Z_n$, où p est un entier inférieur à n,
• appliquer la représentation $R_p$ au modèle d'apprentissage automatique entraîné,
• recevoir, en provenance du modèle d'apprentissage automatique, une ou plusieurs représentations prédites $R_{p-q}^*$ de la région d'examen, chacune desdites une ou plusieurs représentations prédites $R_{p+q}^*$ représentant la région d'examen dans l'état $Z_{p+q}^*$, où q est un indice allant de 1 à m, où m est un nombre entier qui est inférieur à n-1 ou est égal à n-1 ou est supérieur à n-1,
• fournir en sortie et/ou mémoriser et/ou transmettre lesdites une ou plusieurs représentations prédites $R_{p+q}^*$.

3. Procédé selon la revendication 2, dans lequel chaque représentation prédite $R_{p+q}^*$ générée par le modèle d'apprentissage automatique est renvoyée au modèle d'apprentissage automatique entraîné afin de générer une représentation prédite $R_{p+q+1}^*$.

4. Procédé selon la revendication 3, dans lequel m se situe dans la plage de n à n+2.

5. Procédé selon l'une quelconque des revendications 1 à 4, dans lequel les représentations de référence et les représentations prédites et la représentation $R_p$ et les représentations prédites $R_{p+q}^*$ et $R_{p+q+1}^*$ sont des images CT, des images IRM ou des images ultrasonores.

6. Procédé selon la revendication 1, dans lequel la fonction d'erreur totale est donnée par la formule suivante :

$$LV = \sum_{j=2}^{n} w_j \cdot LF_j$$

où LV est la valeur d'erreur totale, où $LF_j$ sont les fonctions d'erreur permettant de calculer les valeurs d'erreur pour les écarts entre la représentation de référence $R_j$ et la représentation prédite $R_j^*$, et $w_j$ sont les facteurs de pondération, où j est un indice allant de 2 à n.

7. Procédé selon l'une quelconque des revendications 1 à 6, dans lequel chaque représentation $R_{p+q}^*$ est calculée selon la formule suivante :

$$\mathbf{M}^q (R_1) = R_{1+q}^*$$

où M représente le modèle d'apprentissage automatique, où $M^q$ désigne l'application q fois du modèle d'apprentissage automatique, et q est un entier qui peut prendre les valeurs 1 à n-1.

8. Procédé selon l'une quelconque des revendications 1 à 7, dans lequel la séquence d'états comprend un ou plusieurs des états suivants :

- un premier état de la région d'examen à un premier instant avant l'application d'un agent de contraste,
- un deuxième état de la région d'examen à un deuxième instant, après l'application de l'agent de contraste,
- un troisième état de la région d'examen à un troisième instant, après l'application de l'agent de contraste,
- un quatrième état de la région d'examen à un quatrième instant, après l'application de l'agent de contraste,
- un cinquième état de la région d'examen à un cinquième instant, après l'application de l'agent de contraste,

le premier instant, le deuxième instant, le troisième instant, le quatrième instant et le cinquième instant formant une séquence temporelle.

9. Système informatique comprenant

- une unité d'entrée,
- une unité de commande et de calcul, et
- une unité de sortie,

l'unité de commande et de calcul étant configurée pour

- recevoir une représentation $R_p$ d'une région d'examen, dans laquelle la représentation $R_p$ représente la région d'examen dans un état $Z_p$ d'une séquence d'états $Z_1$ à $Z_n$, où p est un entier inférieur à n, où n est un entier supérieur à deux, la région d'examen étant le foie ou une partie du foie d'un être humain, la représentation $R_p$ étant une image radiologique,
- appliquer la représentation $R_p$ à un modèle d'apprentissage automatique entraîné,

   o le modèle d'apprentissage automatique entraîné ayant été entraîné sur la base de données d'apprentissage, pour générer, à partir d'une première représentation de référence $R_1$, un nombre n-1 de représentations prédites $R_2^*$ à $R_n^*$,
   o la première représentation de référence $R_1$ représentant la région d'examen dans un premier état $Z_1$ et chaque représentation prédite $R_j^*$ représentant la région d'examen dans l'état $Z_j$, où j est un indice allant de 2 à n,
   o la génération de la représentation prédite $R_2^*$ s'effectuant au moins proportionnellement sur la base de la représentation de référence $R_1$ et la génération de chaque représentation prédite ultérieure $R_k^*$ s'effectuant au moins proportionnellement sur la base de la représentation prédite $R_{k-1}^*$, où k est un indice allant de 3 à n,
   o la représentation prédite $R_2^*$ étant générée en fournissant en entrée la représentation de référence $R_1$ au modèle pendant l'entraînement, et chaque représentation prédite suivante $R_k^*$ étant générée en fournissant en entrée la représentation prédite $R_{k-1}^*$ au modèle pendant l'entraînement,
   o l'entraînement du modèle d'apprentissage automatique comprenant en outre les étapes consistant à :

      - pour chaque représentation prédite $R_j^*$ : calculer une valeur d'erreur pour une paire constituée d'une représentation de référence $R_j$ et de la représentation prédite $R_j^*$ à l'aide d'une fonction d'erreur, où j est un indice allant de 2 à n,
      - calculer une valeur d'erreur totale à l'aide d'une fonction d'erreur totale, la fonction d'erreur totale étant une fonction des valeurs d'erreur dans lesquelles les valeurs d'erreur sont pondérées par des facteurs de pondération,
      - modifier les paramètres du modèle d'apprentissage automatique de telle sorte que la valeur d'erreur totale soit réduite à un minimum défini,

- recevoir du modèle d'apprentissage automatique une ou plusieurs représentations prédites $R_{p-q}^*$ de la région d'examen, chacune desdites une ou plusieurs représentations prédites $R_{p+q}^*$ représentant la région d'examen dans l'état $Z_{p+q}^*$, où q est un indice allant de 1 à m, où m est un nombre entier qui est inférieur à n-1 ou est égal à n-1 ou est supérieur à n-1,
  fournir en sortie et/ou mémoriser et/ou transmettre lesdites une ou plusieurs représentations prédites $R_{p+q}^*$ à un système informatique séparé,
  la séquence d'états comprenant un ou plusieurs des états suivants :

      - le foie ou une partie de celui-ci avant l'application d'un agent de contraste hépatobiliaire,
      - le foie ou une partie de celui-ci pendant la phase artérielle après l'application de l'agent de contraste hépatobiliaire,
      - le foie ou une partie de celui-ci pendant la phase veineuse portale après l'application de l'agent de contraste hépatobiliaire,
      - le foie ou une partie de celui-ci pendant la phase de transition après l'application de l'agent de contraste hépatobiliaire,
      - le foie ou une partie de celui-ci pendant la phase hépatobiliaire après l'application de l'agent de contraste hépatobiliaire.

**10.** Produit programme d'ordinateur comprenant un programme d'ordinateur qui peut être chargé dans une mémoire vive d'un système d'ordinateur et qui, dans celui-ci, amène le système d'ordinateur à mettre en œuvre les étapes suivantes :

- recevoir une représentation $R_p$ d'une région d'examen, la représentation $R_p$ représentant la région d'examen dans un état $Z_p$ d'une séquence d'états $Z_1$ à $Z_n$, où p est un entier inférieur à n, où n est un entier supérieur à deux, la région d'examen étant le foie ou une partie du foie d'un être humain, la représentation $R_p$ étant une image

radiologique,

• appliquer la représentation $R_p$ à un modèle d'apprentissage automatique entraîné,

   o le modèle d'apprentissage automatique entraîné ayant été entraîné sur la base de données d'apprentissage, pour générer, à partir d'une première représentation de référence $R_1$, un nombre n-1 de représentations prédites $R_2^*$ à $R_n^*$,
   o la première représentation de référence $R_1$ représentant la région d'examen dans le premier état $Z_1$ et chaque représentation prédite $R_j^*$ représentant la région d'examen dans l'état $Z_j$, où j est un indice allant de 2 à n,
   o la génération de la représentation prédite $R_2^*$ s'effectuant au moins proportionnellement sur la base de la représentation de référence $R_1$ et la génération de chaque représentation prédite ultérieure $R_k^*$ s'effectuant au moins proportionnellement sur la base de la représentation prédite $R_{k-1}^*$, où k est un indice allant de 3 à n,
   o la représentation prédite $R_2^*$ étant générée en fournissant en entrée la représentation de référence $R_1$ au modèle pendant l'entraînement, et chaque représentation prédite suivante $R_k^*$ étant générée en fournissant en entrée la représentation prédite $R_{k-1}^*$ au modèle pendant l'entraînement,
   o l'entraînement du modèle d'apprentissage automatique comprenant en outre les étapes consistant à :

      - pour chaque représentation prédite $R_j^*$ : calculer une valeur d'erreur pour une paire constituée d'une représentation de référence $R_j$ et de la représentation prédite $R_j^*$ à l'aide d'une fonction d'erreur, où j est un indice allant de 2 à n,
      - calculer une valeur d'erreur totale à l'aide d'une fonction d'erreur totale, la fonction d'erreur totale étant une fonction des valeurs d'erreur dans lesquelles les valeurs d'erreur sont pondérées par des facteurs de pondération,
      - modifier les paramètres du modèle d'apprentissage automatique de telle sorte que la valeur d'erreur totale soit réduite à un minimum défini,

• recevoir, en provenance du modèle d'apprentissage automatique, une ou plusieurs représentations prédites $R_{p-q}^*$ de la région d'examen, chacune desdites une ou plusieurs représentations prédites $R_{p+q}^*$ représentant la région d'examen dans l'état $Z_{p+q}^*$, où q est un indice allant de 1 à m, où m est un nombre entier qui est inférieur à n-1 ou est égal à n-1 ou est supérieur à n-1,

• fournir en sortie et/ou mémoriser et/ou transmettre lesdites une ou plusieurs représentations prédites $R_{p+q}^*$, la séquence d'états comprenant un ou plusieurs des états suivants :

   • le foie ou une partie de celui-ci avant l'application d'un agent de contraste hépatobiliaire,
   • le foie ou une partie de celui-ci pendant la phase artérielle après l'application de l'agent de contraste hépatobiliaire,
   • le foie ou une partie de celui-ci pendant la phase veineuse portale après l'application de l'agent de contraste hépatobiliaire,
   • le foie ou une partie de celui-ci pendant la phase de transition après l'application de l'agent de contraste hépatobiliaire,
   • le foie ou une partie de celui-ci pendant la phase hépatobiliaire après l'application de l'agent de contraste hépatobiliaire.

11. Utilisation d'un agent de contraste dans un procédé d'examen radiologique, le procédé d'examen radiologique comprenant les étapes suivantes :

   • recevoir une représentation $R_p$ d'une région d'examen, la représentation $R_p$ représentant la région d'examen avant ou après l'application de l'agent de contraste dans un état $Z_p$ d'une séquence d'états $Z_1$ à $Z_n$, où p est un entier inférieur à n, où n est un entier supérieur à deux, la région d'examen étant le foie ou une partie du foie d'un être humain, la représentation $R_p$ étant une image radiologique,
   • appliquer la représentation $R_p$ à un modèle d'apprentissage automatique entraîné,

      o le modèle d'apprentissage automatique entraîné ayant été entraîné sur la base de données d'apprentissage, pour générer, à partir d'une première représentation de référence $R_1$, un nombre n-1 de représentations prédites $R_2^*$ à $R_n^*$,
      o la première représentation de référence $R_1$ représentant la région d'examen dans un premier état $Z_1$ et chaque représentation prédite $R_j^*$ représentant la région d'examen dans l'état $Z_j$, où j est un indice allant de 2 à n,

o la génération de la représentation prédite $R_2^*$ s'effectuant au moins proportionnellement sur la base de la représentation de référence $R_1$ et la génération de chaque représentation prédite ultérieure $R_k^*$ s'effectuant au moins proportionnellement sur la base de la représentation prédite $R_{k-1}^*$, où k est un indice allant de 3 à n,

o la représentation prédite $R_2^*$ étant générée en fournissant en entrée la représentation de référence $R_1$ au modèle pendant l'entraînement, et chaque représentation prédite suivante $R_k^*$ étant générée en fournissant en entrée la représentation prédite $R_{k-1}^*$ au modèle pendant l'entraînement,

o l'entraînement du modèle d'apprentissage automatique comprenant en outre les étapes consistant à :

- pour chaque représentation prédite $R_j^*$ : calculer une valeur d'erreur pour une paire constituée d'une représentation de référence $R_j$ et de la représentation prédite $R_j^*$ à l'aide d'une fonction d'erreur, où j est un indice allant de 2 à n,
- calculer une valeur d'erreur totale à l'aide d'une fonction d'erreur totale, la fonction d'erreur totale étant une fonction des valeurs d'erreur dans lesquelles les valeurs d'erreur sont pondérées par des facteurs de pondération,
- modifier les paramètres du modèle d'apprentissage automatique de telle sorte que la valeur d'erreur totale soit réduite à un minimum défini,

• recevoir, en provenance du modèle d'apprentissage automatique, une ou plusieurs représentations prédites $R_{p-q}^*$ de la région d'examen, chacune desdites une ou plusieurs représentations prédites $R_{p+q}^*$ représentant la région d'examen dans l'état $Z_{p+q}^*$, où q est un indice allant de 1 à m, où m est un nombre entier qui est inférieur à n-1 ou est égal à n-1 ou est supérieur à n-1,

• fournir en sortie et/ou mémoriser et/ou transmettre lesdites une ou plusieurs représentations prédites $R_{p+q}^*$, la séquence d'états comprenant un ou plusieurs des états suivants :

• le foie ou une partie de celui-ci avant l'application d'un agent de contraste hépatobiliaire,
• le foie ou une partie de celui-ci pendant la phase artérielle après l'application de l'agent de contraste hépatobiliaire,
• le foie ou une partie de celui-ci pendant la phase veineuse portale après l'application de l'agent de contraste hépatobiliaire,
• le foie ou une partie de celui-ci pendant la phase de transition après l'application de l'agent de contraste hépatobiliaire,
• le foie ou une partie de celui-ci pendant la phase hépatobiliaire après l'application de l'agent de contraste hépatobiliaire.

12. Kit comprenant un agent de contraste et un produit programme d'ordinateur, le produit programme d'ordinateur comprenant un programme d'ordinateur qui peut être chargé dans une mémoire vive d'un système informatique et qui, dans celui-ci, amène le système informatique à mettre en œuvre les étapes suivantes :

• recevoir une représentation $R_p$ d'une région d'examen, la représentation $R_p$ représentant la région d'examen avant ou après l'application de l'agent de contraste dans un état $Z_p$ d'une séquence d'états $Z_1$ à $Z_n$, où p est un entier inférieur à n, où n est un entier supérieur à deux, la région d'examen étant le foie ou une partie du foie d'un être humain, la représentation $R_p$ étant une image radiologique,

• appliquer la représentation $R_p$ à un modèle d'apprentissage automatique entraîné,

o le modèle d'apprentissage automatique entraîné ayant été entraîné sur la base de données d'apprentissage, pour générer, à partir d'une première représentation de référence $R_1$, un nombre n-1 de représentations prédites $R_2^*$ à $R_n^*$,

o la première représentation de référence $R_1$ représentant la région d'examen dans un premier état $Z_1$ et chaque représentation prédite $R_j^*$ représentant la région d'examen dans l'état $Z_j$, où j est un indice allant de 2 à n,

o la génération de la représentation prédite $R_2^*$ s'effectuant au moins proportionnellement sur la base de la représentation de référence $R_1$ et la génération de chaque représentation prédite ultérieure $R_k^*$ s'effectuant au moins proportionnellement sur la base de la représentation prédite $R_{k-1}^*$, où k est un indice allant de 3 à n,

o la représentation prédite $R_2^*$ étant générée en fournissant en entrée la représentation de référence $R_1$ au modèle pendant l'entraînement, et chaque représentation prédite suivante $R_k^*$ étant générée en fournissant en entrée la représentation prédite $R_{k-1}^*$ au modèle pendant l'entraînement,

o l'entraînement du modèle d'apprentissage automatique comprenant en outre les étapes consistant à :

- pour chaque représentation prédite $R_j^*$ : calculer une valeur d'erreur pour une paire constituée d'une représentation de référence $R_j$ et de la représentation prédite $R_j^*$ à l'aide d'une fonction d'erreur, où j est un indice allant de 2 à n,
- calculer une valeur d'erreur totale à l'aide d'une fonction d'erreur totale, la fonction d'erreur totale étant une fonction des valeurs d'erreur dans lesquelles les valeurs d'erreur sont pondérées par des facteurs de pondération,
- modifier les paramètres du modèle d'apprentissage automatique de telle sorte que la valeur d'erreur totale soit réduite à un minimum défini,

• recevoir, en provenance du modèle d'apprentissage automatique, une ou plusieurs représentations prédites $R_{p-q}^*$ de la région d'examen, chacune desdites une ou plusieurs représentations prédites $R_{p+q}^*$ représentant la région d'examen dans l'état $Z_{p+q}^*$, où q est un indice allant de 1 à m, où m est un nombre entier qui est inférieur à n-1 ou est égal à n-1 ou est supérieur à n-1,

• fournir en sortie et/ou mémoriser et/ou transmettre lesdites une ou plusieurs représentations prédites $R_{p+q}^*$, la séquence d'états comprenant un ou plusieurs des états suivants :

• le foie ou une partie de celui-ci avant l'application d'un agent de contraste hépatobiliaire,
• le foie ou une partie de celui-ci pendant la phase artérielle après l'application de l'agent de contraste hépatobiliaire,
• le foie ou une partie de celui-ci pendant la phase veineuse portale après l'application de l'agent de contraste hépatobiliaire,
• le foie ou une partie de celui-ci pendant la phase de transition après l'application de l'agent de contraste hépatobiliaire,
• le foie ou une partie de celui-ci pendant la phase hépatobiliaire après l'application de l'agent de contraste hépatobiliaire.

**(a)**

**(b)**

Fig. 1

Fig. 2

Fig. 3

Fig. 4

Fig. 5

$q$-1 Iterationen

$R_p$
$Z_p$

M

S

$R_{p+q}$
$Z_{p+q}$

**Fig. 6**

(10)

(20)

(30)

(1)

**Fig. 7**

(1)

(31)

(11)

(32)

(21)

(12)

(22)

(33)

(40)

Fig. 8

**IN DER BESCHREIBUNG AUFGEFÜHRTE DOKUMENTE**

**In der Beschreibung aufgeführte Patentdokumente**

- WO 2021052896 A1

- US 6039931 A

**In der Beschreibung aufgeführte Nicht-Patentliteratur**

- **L. ZHANG et al.** offenbaren räumlich-zeitliche Faltungs-LSTMs für die Vorhersage von Tumorwachstum durch Lernen von 4D-Längsschnittdaten von Patienten (Spatio-Temporal Convolutional LSTMs for Tumor Growth Prediction by Learning 4D Longitudinal Patient Data. *IEEE Trans Med Imaging.*, April 2020, vol. 39 (4), 1114-1126

- **P. LUC et al.** offenbaren Verfahren zur Vorhersage zukünftiger semantischer Segmentierungen (Predicting Deeper into the Future of Semantic Segmentation. *Proceedings - 2017 IEEE International Conference on Computer Vision, ICCV*, 2017, 648-657

- **A. S. L. JASCINTH et al.** Contrast Agents in computed tomography: A Review. *Journal of Applied Dental and Medical Sciences*, 2016, vol. 2 (2), 143-149

- **H. LUSIC et al.** X-ray-Computed Tomography Contrast Agents. *Chem. Rev.*, 2013, vol. 113 (3), 1641-1666, https://www.radiology.wisc.edu/wp-content/uploads/2017/10/contrast-agents-tutorial.pdf

- **M. R. NOUGH**. Radiographie and magnetic resonances contrast agents Essentials and tips, for safe practices. *World J Radiol.*, 28 September 2017, vol. 9 (9), 339-349

- **L. C. ABONYI et al.** Intravascular Contrast Media in Radiography: Historical Development & Review ofRisk Factors for Adverse Reactions,. *South American Journal of Clinical Research*, 2016, vol. 3 (1), 1-10

- *ACR Manual on Contrast Media*, 2020, ISBN 978-1-55903-012-0

- **A. IGNEE et al.** Ultrasound contrast agents. *Endosc Ultrasound.*, November 2016, vol. 5 (6), 355-362

- *J. Magn. Reson. Imaging*, 2012, vol. 35 (3), 492-511

- *Clujul Medical*, 2015, vol. 88 (4), 438-448

- *Journal of Hepatology*, 2019, vol. 71, 534-542, http://dx.doi.org/10.1016/j.jhep.2019.05.005

- **R. MECHREZ et al.** The Contextual Loss for Image Transformation with Non-Aligned Data,. *arxiv:1803.02077v4*, 2018

- **H. ZHAO et al.** Loss Functions for Image Restoration with Neural Networks,. *arxiv:1511.08861v3*, 2018

- U-net: Convolutional networks for biomedical image segmentation. **O. RONNEBERGER et al.** International Conference on Medical image computing and computer-assisted intervention. Springer, 2015, 234-241

- **M.-Y. LIU et al.** Generative Adversarial Networks for Image and Video Synthesis: Algorithms and Applications. *arXiv:2008.02793*

- **J. HENRY et al.** *Pix2Pix GAN for Image-to-Image Translation,*

- **Y. GAO et al.** *Fully convolutional structured LSTM networks for joint 4D medical image segmentation*

- **D. KARIMI et al.** Convolution-Free Medical Image Segmentation using Transformers. *arXiv:2102.13645 [eess.IV*